(19) **Europäisches Patentamt**
**European Patent Office**
**Office européen des brevets**

(11) **EP 3 897 377 B1**

(12) **EUROPEAN PATENT SPECIFICATION**

(45) Date of publication and mention
of the grant of the patent:
**19.11.2025 Bulletin 2025/47**

(21) Application number: **19832014.5**

(22) Date of filing: **13.12.2019**

(51) International Patent Classification (IPC):
*A61B 5/05* (2021.01)   *G01V 3/10* (2006.01)
*A61B 5/053* (2021.01)   *A61B 5/00* (2006.01)

(52) Cooperative Patent Classification (CPC):
**A61B 5/0537; A61B 5/7264; G01V 3/10;**
A61B 5/0522

(86) International application number:
**PCT/EP2019/085026**

(87) International publication number:
**WO 2020/126878 (25.06.2020 Gazette 2020/26)**

(54) **INDUCTIVE SENSING SYSTEM AND METHOD**

SYSTEM UND VERFAHREN ZUR INDUKTIVEN MESSUNG

SYSTÈME ET PROCÉDÉ DE DÉTECTION INDUCTIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2018 EP 18213488**

(43) Date of publication of application:
**27.10.2021 Bulletin 2021/43**

(73) Proprietor: **Koninklijke Philips N.V.**
**5656 AG Eindhoven (NL)**

(72) Inventors:
• **PEETERS, Wouter, Herman**
  **5656 AE Eindhoven (NL)**
• **KLEIJNEN, Mark, Peter, Paul**
  **5656 AE Eindhoven (NL)**
• **STEUNEBRINK, Tim, Patrick**
  **5656 AE Eindhoven (NL)**
• **DOODEMAN, Gerardus, Johannes, Nicolaas**
  **5656 AE Eindhoven (NL)**
• **BEZEMER, Rick**
  **5656 AE Eindhoven (NL)**
• **VERNOOIJ, Carlijn, Andrea**
  **5656 AE Eindhoven (NL)**
• **GIGI, Ercan, Ferit**
  **5656 AE Eindhoven (NL)**

(74) Representative: **Philips Intellectual Property &
Standards
High Tech Campus 52
5656 AG Eindhoven (NL)**

(56) References cited:
WO-A1-2018/127482   US-A1- 2008 007 275
US-A1- 2010 219 841   US-A1- 2010 305 499
US-A1- 2012 065 617   US-A1- 2015 374 292
US-A1- 2016 223 483

**Description**

FIELD OF THE INVENTION

The present invention relates to an inductive sensing system and method, in particular for sensing electromagnetic signals emitted from a body responsive to propagation into the body of electromagnetic excitation signals.

BACKGROUND OF THE INVENTION

There is commonly a need to measure mechanical movements and dynamical changes of internal bodily structures such as the heart, the lungs, or arteries. For instance, it is useful to measure the cyclically varying internal volume or dimensions of chambers of the heart, or of the lungs, or of mechanical activity of arteries, e.g. changing arterial volumes over heart cycles.

Sensors which measure mechanical activity are sometimes called kymographic sensors (kymographic biometric sensors in the clinical field). Some examples of kymographic biometric sensors are accelerometer-based biosensors, transthoracic impedance biosensors, radar-based biosensors, capacitive sensors, and photoplethysmography (PPG) sensors.

Magnetic inductive sensors also have the potential to be used as biometric sensors for sensing mechanical activity. The working principle of inductive sensing is based on Faraday's law. An oscillating primary magnetic field is generated by a generating loop antenna, and this induces, via Faraday's law, eddy currents in the tissue irradiated by the signals. The eddy currents generate a secondary magnetic field. The total magnetic field is then a superposition of the primary magnetic field and the secondary magnetic field. The changes induced in electrical characteristics of the generating antenna (the antenna current) can be measured and these used to deduce characteristics of the secondary field, and thus the stimulated tissue.

Inductive sensing provides the potential for simple contactless measurements of heart and lung mechanical activity, or the mechanical activity of a blood vessel such as the radial artery in a human arm.

A very significant disadvantage of kymographic biosensors in general (including inductive sensors and other types of sensors) is that it is currently very different to distinguish sensed signals originating from different physiological sources or activities.

For example, it is very difficult to tell apart, within a single composite returned signal, signal elements relating to mechanical activity of the heart (e.g. heart pulse) and elements relating to mechanical activity of the lungs (e.g. respiration).

In known systems, this is often performed by assuming that the heart rate is larger than the breathing rate. In this way, the different signal components can be distinguished and separated based on frequency.

However, the clinically possible ranges for the heart rate and pulse rate overlap. For example, frequencies of a particularly high breathing rate overlap with frequencies of a particularly low pulse rate, and vice versa. Therefore, a sensed (high) breathing rate can be incorrectly interpreted by state of the art systems as a low heart rate, and vice versa, leading to erroneous clinical diagnoses and interventions.

For example, in neonatal monitoring, apneas are frequently not detected by impedance based measurements, for the reason that the heart contractions characteristic of this condition are interpreted incorrectly as the patient's breathing rate.

A further, related, problem with known systems is that motion artefacts are also difficult to distinguish from true biophysical signals, such as a pulse signal or a breathing signal. Distinguishing motion artefacts from true signals is again often performed by assuming that the frequency of the artefact is different from the frequency of the biometric signal and/or that the waveform of the artefact is different (e.g. in shape characteristics) from the waveform of the biometric signal. These methods however are often unsatisfactory, because both the frequency and the waveform of the signal artefact are often similar to the frequency and waveform of the biometric signal.

For example, if a patient's step frequency during walking is close to the pulse rate, (which is commonly the case) then measurements of the pulse frequency become unreliable. Additionally, even in the case that the frequencies are different, the patient's step frequency may nonetheless be interpreted as the pulse frequency due for instance to a very similar waveform of the two. This results in an incorrect measurement of the heart rate.

WO2018/127482 A1 discloses a magnetic inductive sensing system for sensing electromagnetic signals emitted from a body in response to electromagnetic excitation signals applied to the body. The electromagnetic signals are generated and sensed by the same loop resonator which comprises a single-turn loop antenna and a tuning capacitor.

US 2015/374292 A1 devices, systems and methods that use volumetric integral phase-shift spectroscopy ("VIPS") to monitor changes in fluids in the brain or other parts of the body.

An improved approach to inductive sensing is therefore required which is capable of more reliably distinguishing different physiological signals both from one another and from signal artefacts.

## SUMMARY OF THE INVENTION

The invention is defined by the claims.

According to examples in accordance with an aspect of the invention, there is provided an inductive sensing system for sensing electromagnetic signals returned from a body responsive to application of electromagnetic excitation signals into said body, the system comprising:

a resonator circuit comprising a loop antenna;

a signal generation means adapted to excite the loop antenna to generate the electromagnetic excitation signals,

a signal sensing means adapted to sense said returned signals from the body using the loop antenna, based on detecting variations in electrical characteristics of the resonator circuit;

and a controller, wherein the controller is adapted to:

detect from the sensed returned signals measures indicative of both a real and an imaginary part of an additional inductance component induced in the resonator circuit by the received electromagnetic signals, and

using the detected measures indicative of real and imaginary inductance components, extract from the returned electromagnetic signals one or more individual signal components corresponding to different respective physiological sources, the extraction being based on relative magnitudes of said detected real and imaginary inductance components added to the resonator circuit by the returned signals.

The extracted signal components are separate, individual, signal components, each corresponding to a different respective known physiological source in the body. In other words each signal component is an isolated signal component, separated from any other signal component corresponding to a different physiological source.

Embodiments of the invention are based on the insight that signals originating from different physiological or anatomical sources in the body exhibit different characteristic relative magnitudes of real and imaginary parts of an additional inductance component induced in a sensing antenna when a signal from that source reaches the antenna.

The proposed approach is based on measuring, from changes in electrical characteristics of the antenna, measures related to or indicative of both a real and an imaginary part of an additional inductance component induced at the antenna loop (or resonator circuit) by an overall composite signal received back from the body.

In some embodiments, an independent component analysis method may be applied to the signals representative of the detected measures indicative of real and imaginary components in order to determine and extract the separate individual signal components corresponding to different physiological sources. This could be done in an 'on-line' manner, such that detected signals representative of said measures indicative of real and imaginary added inductance components are processed in real time with the independent component analysis procedure, and the different component physiological signals separated and extracted based on this.

In accordance with a further set of embodiments, a dataset of known characteristic relative magnitudes of real and imaginary parts of corresponding inductance signals (or measures related thereto) for different specific physiological signal sources can be consulted, and these can be used to extract from the composite signal the signal elements corresponding to different physiological sources.

For example, the detected measures indicative of real and imaginary additional inductance parts and the stored measures or information relating to different characteristic relative magnitudes of real and imaginary inductance parts can be thought of or understood as vectors in the complex plane for the additional induced inductance component. Extraction of the different signal components for different physiological sources can realized by effectively projecting the stored characteristic vectors onto the measured vector of real and imaginary parts. Only that part of the composite signal having the same directionality in the complex plane as one of the stored characteristic vectors is output from this projection. This part then corresponds to the signal component originating from the physiological source corresponding to that vector.

In general, when a loop antenna is brought into proximity with a body, the inductance L acquires an additional reflected inductance component, $L_r$, arising due to eddy currents induced in the stimulated body as a result of application of the excitation signals. These eddy currents in turn effectively make a contribution to the inductance of the loop antenna, due to the generation of a secondary time-varying magnetic flux. These eddy-current fluxes combine with the primary flux of the antenna, resulting in a greater induced back-EMF in the antenna, and hence a larger measurable effective inductance.

The added component of inductance arising from the eddy currents may be referred to synonymously in this disclosure as 'reflected inductance', $L_r$. The total inductance $L_t$ of the coil antenna may be expressed as:

$$L_t = L_0 + L_r \tag{1}$$

where $L_0$ is the self-inductance of the antenna in free space and $L_r$ is the reflected inductance caused by the proximity of the stimulated body or medium.

The reflected inductance is closely related to the reflected impedance $Z_r$. The relationship is $L_r = Z_r / i\omega$, where $\omega$ is the radial frequency of the electromagnetic excitation signals (the time-varying field applied to the body).

In general, the reflected inductance, $L_r$, is complex, and can be expressed as

$$L_r = L'_r + iL''_r \qquad (2)$$

where $L'_r$ is related to a reactive impedance of the antenna and $L''_r$ is related to resistive impedance of the antenna.

The addition of the reflected component of inductance $L_r$ leads to a detuning of the characteristics of the antenna (or resonator circuit). In particular, both the natural radial frequency of the coil antenna circuit and the damping factor of the coil antenna circuit change.

In particular, the real part of the additional inductance component, $L_r$, manifests itself in the frequency of the resonator circuit or antenna. The imaginary part of the additional inductance component manifests in the amplitude of the resonator circuit. Hence, in one set of examples, the real and imaginary parts of the additional inductance components may be detected based on measuring changes in the frequency and amplitude of the resonator circuit (current) respectively. Other approaches are possible however, as will be discussed below.

The resonator circuit referred to above is for coupling with returned electromagnetic signals for the purpose of measuring or detecting those signals.

The signal sensing is this approach is thus performed simultaneously with signal generation.

The resonator circuit may comprise a capacitor component electrically coupled with the loop antenna.

The resonator circuit has an electrical resonance frequency. This may be defined at least in part by a provided capacitor component comprised by the circuit.

As mentioned above, in accordance with one set of embodiments, the extraction of the individual signal components may be based on application of an Independent Component Analysis procedure to the detected measures indicative of real and imaginary inductance components.

In accordance with a further set of embodiments, the extraction is based on consulting a dataset of information indicative of known relative magnitudes of real and imaginary added inductance components for different physiological signal sources.

In particular, in this set of embodiments, the system may be configured to

access a dataset which stores, for signals from a plurality of different known physiological sources within said body, information indicative of characteristic relative magnitudes of real and imaginary inductance components added to a resonator circuit by a signal received from said physiological source; and
using the detected measures indicative of real and imaginary inductance components, and based on consulting said dataset, extract from the returned electromagnetic signals the one or more individual signal components corresponding to different respective known physiological sources.

The dataset stores information indicative of relative magnitudes of real and imaginary parts of inductance components added by signals originating from different physiological sources. By relative magnitudes may be meant relative to one another, e.g. the magnitudes are normalized, such that their sum, or the sum of their squares is one.

The stored information indicative of characteristic relative real and imaginary part magnitudes can take many different particular forms or representations. In some examples, the relative magnitudes may be stored in numerical form. In some examples, the relative magnitudes may be stored in vector form, e.g. with each relative magnitude forming one vector element or component.

In some examples, there may be stored a different measure, this measure being indicative of or related to, or proportional to for instance the relative magnitudes of the real and imaginary inductance parts. For instance, in some examples, the physiological signal may be divided by each of the real and imaginary parts, and these quotients or ratios stored. In some examples, the complex argument or phase of the additional (complex) inductance component induced by a signal received from the given physiological source may be stored. The argument essentially indicates a direction in the complex plane of additional inductance component, from which relative magnitudes of real and imaginary parts of the additional inductance part directly follow.

According to one set of examples, the stored information for each physiological signal source may take the form of a vector or point in a complex plane for the additional inductance component added to the sensing resonator circuit by a signal received from the physiological source.

The vector or point may be referred to in this disclosure as a characteristic vector or point. The vector or point is representative of an additional inductance component added to a resonator circuit by a signal received from the respective physiological source.

The characteristic vector may be normalized in some examples (i.e. the vector may be a unit vector in the complex plane).

Instead of a vector, the stored information may in other examples simply take the form of a representation of a direction of such a vector within the complex plane.

In further examples, the stored information may take the form of a characteristic argument or phase of an additional inductance component induced by a signal received from the physiological source (as noted above).

According to one set of examples, said characteristic relative magnitudes represented by the stored information are weighted so as to provide a multiplicative mapping between the detected measures indicative the real and imaginary parts of the additional sensed inductance component and an extracted signal component for a given physiological source.

This means that the stored measure indicative of the real part and the stored measure indicative of the imaginary part may be weighted such that multiplying each with the corresponding real or imaginary part of the measured additional inductance component results directly in a signal output representative of a signal component originating from the corresponding physiological source.

According to one set of examples, said stored information for each physiological signal source may comprise a characteristic vector having:

> a first vector component indicative of a ratio between a physiological signal received from said source and a measure indicative of the real part of a received additional inductance component for said signal, and
>
> a second vector component indicative of a ratio between a physiological signal received from said source and a measure indicative of the imaginary part of a received additional inductance component for said signal.

Here, the characteristic relative magnitudes stored in the dataset are determined in advance of performing the actual inductive signal measurements and extraction process. The physiological signal received from the source may mean for instance the true physiological signal in the units of the physiological phenomenon measured. For example, a lung volume signal may be in units of liters.

The physiological signal means the physiological parameter or quantity which is being measured, i.e. which is represented by the additional inductance component added to the resonator circuit. Hence the first vector component may be indicative of a ratio between a physiological parameter or quantity related to the physiological source and a measure indicative of the real part of an additional inductance component added to a resonator circuit by a signal received from said physiological source, and

the second vector component may be indicative of a ratio between a physiological parameter or quantity related to said physiological source and a measure indicative of the imaginary part of an additional inductance component added to a resonator circuit by a signal received from said physiological source.

The first and second vector components correspond to an actual measured signal divided respectively by the corresponding measured real and imaginary components of the resulting sensed additional inductance component in the resonator circuit.

Each vector encompasses therefore an element based on a magnitude of the relevant physiological parameter measurement divided by the real part of the corresponding additional received inductance component, and an element based on the magnitude the relevant physiological parameter measurement divided by the imaginary part of the corresponding additional inductance component.

Where the stored measure or information relating to relative real and imaginary inductance parts is stored or represented in the form of a (characteristic) vector, the system may be adapted to:

extract the one or more signal components from different known physiological sources based on taking an inner product of the stored vector corresponding to each physiological source and a further vector, the further vector representative of said measures indicative of real and imaginary parts of the sensed returned electromagnetic signal(s). The vector elements of the further vector may be the real and imaginary parts of the measured additional inductance component, $L_r$, induced in the resonator circuit, i.e. ($Re\{L_r\}$, $Im\{L_r\}$) for instance. The vector elements may each be signals representative of said real and imaginary components as a function of time.

Optionally a low pass filter may be applied to components of said further vector in advance of performing the inner product in order to remove a baseline and/or a band-pass filter may be applied to said further vector in advance of performing the inner product. The band pass filter may remove a baseline in addition to high frequency noise components for example.

The signal sensing means referred to above may be adapted to detect said measure indicative of the real part of the additional inductance component based on detecting a change in a reactance of the resonator circuit. The real part of the additional inductance component, $L_r$, leads to a modified antenna reactance of magnitude $\omega \cdot Re\{L_r\}$. This will be further explained below.

Additionally or alternatively, the signal sensing means may be adapted to detect said measure indicative of the imaginary part of the additional inductance component based on detecting a change in a resistance of the antenna coil. The additional induced inductance component, $L_r$, leads to a modified antenna resistance of magnitude $-\omega \cdot \text{Im}\{L_r\}$. This will be further explained below.

According to one or more embodiments, the signal sensing means may be adapted to detect said measure indicative of the real part of the additional inductance component based on determining changes in a frequency of the resonator circuit current. As noted above, the real part of the additional inductance component manifests in a modified reactance of the resonator circuit current. This modified reactance leads to a modified resonator frequency. This will be explained further below.

Monitoring changes in the frequency of the resonator circuit provides a very simple, straightforward means of detecting changes in the real part of the additional inductance component induced at the resonator circuit. The measure detected here is therefore the frequency.

Additionally or alternatively, the signal sensing means may be adapted to detect said measure indicative of the imaginary part of the additional inductance component based on determining changes in an amplitude of a current in the antenna coil or resonator circuit (current).

As noted above, the imaginary part of the additional inductance component manifests in a modified resistance of the resonator circuit current. This modified resistance leads to a modified damping of the resonator circuit. This in turn leads to a modified amplitude of the resonator circuit current. This will be explained further below.

Monitoring changes in the amplitude of the resonator circuit (current) provides a very simple, straightforward means of detecting changes in the imaginary part of the additional inductance component induced at the resonator circuit. The measure detected here is therefore the current amplitude.

As noted above, in particular examples, the stored information for each physiological signal source may take the form of a characteristic vector or point in a complex plane for the additional inductance component. In certain examples in such cases

the signal generation means may be adapted to excite the resonator to generate the excitation signals having a radial frequency $\omega$, and

the controller may be adapted in at least one control mode to perform an adjustment of said radial frequency from a first frequency to a second frequency, to thereby increase a degree (e.g. average degree) of orthogonality between vectors formed in the complex plane by said real and imaginary components of the additional induced inductance components for the different physiological sources.

Thus the frequency adjustment is to increase a degree of orthogonality between respective vectors formed in the complex plane by the additional induced inductance components added to the resonator circuit by signals received from the different respective physiological sources.

This set of embodiments is based on the insight that the relative directions in the complex plane of the reflected complex inductance components induced by different physiological sources vary in dependence upon the frequency of the excitation signals. Hence, by adjusting the frequency, the relative angles (i.e. argument angles) in the complex plane between these respective inductance components (as represented as vectors) for different physiological sources varies. In this way, the frequency can be changed from a first frequency, associated with a first orthogonality (e.g. average orthogonality) between the vectors in the complex plane for different physiological signals to a second frequency, associated with a second, greater, orthogonality between the vectors in the complex plane for different physiological sources.

The more orthogonal are the vectors (representative of the different corresponding inductance components) for different physiological sources with respect to one another, the better the signal extraction or separation procedure works. This renders the signal extraction process less prone to motion artefacts and other sources of noise. It is therefore beneficial to increase the orthogonality of the vectors.

In other words, the frequency of the applied excitation signals may be adjusted in order to better orthogonalize the characteristic vectors of the induced reflected inductance $L_r$ components at the antenna for the different physiological sources within the body. Greater orthogonality of these characteristic $L_r$ signals leads to more reliable and robust extraction and separation of signal components for the different physiological sources. It is therefore advantageous to adjust the applied signal frequencies to maximize orthogonality between the characteristic vectors.

The system, e.g. a controller, may monitor the degree of orthogonality of the vectors representative of the different induced inductance components whilst performing the adjustment procedure. A feedback loop can be implemented, such that the frequency adjustment is guided by the monitored vector orthogonality.

According to one or more embodiments, the system may be further adapted to perform a learning procedure comprising determining or updating the stored information for each of the physiological sources. The learning procedure may be performed for example as an initial procedure, for example a calibration or setup procedure, before use of the sensing system for a particular patient. There may be no stored information for the different physiological sources until the learning procedure is performed in some examples. The learning procedure may in some examples comprise a process of learning or determining or updating the fingerprint vectors.

Additionally or alternatively, according to one or more embodiments, the system may be adapted to update said stored information for each physiological source to reflect changes induced in the real and imaginary components of the additional induced inductance components for the different physiological sources by said frequency adjustment.

There are different ways of implementing the learning procedure. According to one or more embodiments, the system may be adapted to record information related to signal characteristics of each extracted signal component, and wherein the system is adapted to perform a learning procedure comprising adjusting the stored information for each of the physiological sources based on the recorded information.

For instance, the system may be adapted to monitor the same signal characteristics in future extracted signal components and to adjust the characteristic measures so as to reduce a variance or increase a uniformity in those characteristics of future output signal components.

The recorded information may relate for instance to shape characteristics of the extracted signal components, or other signal characteristics such as amplitude, frequency composition, baseline, or any other signal characteristics.

The system may comprise a controller configured to perform the above procedure, or the signal processing means may perform the procedure for instance.

According to one or more embodiments, the learning procedure may comprise performing an independent component analysis (ICA) procedure upon the sensed returned signals sensed at the antenna. It may for example comprise performing an ICA procedure upon signals representative of said real and imaginary components of inductance added to the resonator circuit by returned signals. ICA is a known methodology in signal processing but has never been applied in the field of inductive sensing. The method is based on an assumption that returned signals sensed at the resonator circuit antenna (in the form of the additional inductance component) are composite signals formed of a plurality of signal components, each corresponding to a different physiological source. ICA can be applied to determine or extract the underlying physiological signal components in the sensed inductive signals.

In preferred examples, the real and imaginary components of the additional inductance sensed at the antenna are taken each as individual signals, and the ICA procedure applied to these real and imaginary inductance signals to determine the component physiological signals forming them.

In accordance with one or more embodiments, the system may be adapted to implement a multiple frequency drive scheme comprising driving (exciting) the antenna at a plurality of frequencies in a successive manner. It may cycle between a plurality of different frequencies. In accordance with one or more embodiments, the system may be adapted to drive the antenna with a plurality of drive frequencies in a time-multiplexed manner and to sense the returned signals at the antenna at each of said drive frequencies. Time-multiplexed means that the antenna is switched between the different frequencies successively.

This multi-frequency drive scheme thereby permits obtaining in each duty cycle one or more measurement signals for each separate frequency. This thereby increases the number of obtained signals.

The antenna is alternated or cycled through the different drive frequencies in successive manner, one frequency at a time, and inductive sensor measurements taken simultaneously with signal generation at each of the drive frequencies.

According to one or more sets of embodiments, the system may include signal processing means configured to process the extracted one or more signal components and derive one or more physiological parameter measurements based on the signal component(s).

The signal sensing means may be pre-programmed with one or more algorithms for instance to facilitate determination (i.e. quantification) of the physiological parameter measurements.

The signal processing means may be comprised by the signal sensing means in some examples, or the functionality may be facilitated by the signal sensing means.

In other examples, the parameter measurement derivation procedure may be performed by a separate provided controller, or by any other suitable component of the system.

Examples in accordance with a further aspect of the invention provide an inductive sensing method based on sensing electromagnetic signals returned from a body responsive to application of electromagnetic excitation signals into said body, the method comprising:

applying electromagnetic excitation signals into a body using a resonator circuit, the resonator circuit comprising a loop antenna and an electrically coupled capacitor;
sensing said returned signals from the body using the loop antenna, based on detecting variations in electrical characteristics of the resonator circuit;
detecting from the sensed returned signals measures indicative of both a real and an imaginary part of an additional inductance component induced in the resonator circuit by the received electromagnetic signals,
extracting from the returned electromagnetic signals one or more individual signal components from different known physiological sources, based on use of the detected measures indicative of real and imaginary inductance components, the extracting being based on relative magnitudes of said detected real and imaginary inductance components added to the resonator circuit by the returned signals, wherein a controller is adapted to perform the detecting and extracting.

Implementation options and details for each of the above steps may be understood and interpreted in accordance with the explanations and descriptions provided above for the apparatus aspect of the present invention (i.e. the system aspect).

Any of the examples, options or embodiment features or details described above in respect of the apparatus aspect of this invention (i.e. in respect of the system) may be applied or combined or incorporated into the present method aspect of the invention.

In accordance with one or more embodiments, the extraction may be based on performance of an independent component analysis procedure, as discussed above in relation to the system.

In accordance with a further set of embodiments, the method may be based on use of a dataset. In particular, the method may comprise:

accessing a dataset which stores, for signals from a plurality of different known physiological sources within said body, information indicative of characteristic relative magnitudes of real and imaginary inductance components added to a resonator circuit by a signal received from said physiological source; and

extracting from the returned electromagnetic signals one or more individual signal components from different known physiological sources, based on use of the detected measures indicative of real and imaginary inductance components, and based on consulting said dataset.

According to one or more embodiments, the stored information for each physiological signal source may take the form of a vector or point in a complex plane for the additional inductance component added to the sensing resonator circuit by a signal received from the physiological source. The vector or point may be referred to in this disclosure as a characteristic vector or point.

Thus, the stored information for each physiological signal source may take the form of a vector or point in a complex plane, the vector or point representative of an additional inductance component added to a resonator circuit by a signal received from the respective physiological source.

According to one or more embodiments, detecting said measure indicative of the real part of the additional inductance component may be performed based on detecting changes in a frequency of a current in the resonator circuit.

Additionally or alternatively, according to one or more embodiments, detecting said measure indicative of the imaginary part of the additional inductance component is performed based on detecting changes in an amplitude of a current in the resonator circuit.

According to one or more embodiments, the resonator circuit may be controlled to generate excitation signals having a radial frequency $\omega$, and

the method may include in at least one control mode performing an adjustment of said radial frequency from a first frequency to a second frequency, to thereby increase a degree (e.g. average degree) of orthogonality between vectors in the complex plane formed by said real and imaginary components of the additional induced inductance components for the different physiological sources.

In other words, the frequency adjustment is to increase a degree of orthogonality between respective vectors formed in the complex plane by the additional induced inductance components added to the resonator circuit by signals received from the different respective physiological sources.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically depicts application of excitation signals to a body and receipt of returned secondary signals from the body

Fig. 2 illustrates right and left travelling vector potential field components at medium layer interfaces;

Fig. 3 illustrates an example set up of a sensing system and application of excitation signals to a multi-layered medium;

Fig. 4 shows a plot illustrating variation of complex phase of induced reflected inductance at the antenna as a function of location of the signal source;

Fig. 5 shows in block diagram form an example inductive sensing system according to one or more embodiments of the invention;

Fig. 6 schematically depicts the workflow for extraction of signal components originating from different physiological signal sources;

Fig. 7 shows a graph illustrating extracting physiological source signals based on measured inductive sensing signals using an Independent Component Analysis method;

Fig. 8 illustrates directions of example characteristic vectors for reflected inductance signals from two different example physiological sources;

Fig. 9 shows variation of direction of characteristic vectors for reflected inductance signals as a function of changing frequency of applied excitation signals;

Fig. 10 illustrates orthogonal eye is a of characteristic vectors for reflected inductance signals for different physiological sources based on adjusting the frequency of applied excitation signals; and

Fig. 11 shows a block diagram of an example inductive sensing method in accordance with one or more embodiments.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an inductive sensing system adapted to apply electromagnetic excitation signals to a body, the system comprising a resonator circuit incorporating a loop antenna. The system senses signals returned back from the body with the same antenna, based on variation in electrical characteristics of the resonator circuit. The system is configured for separating signals received from different physiological sources within the body. This is performed based on detecting in the resonator circuit electrical characteristics indicative of both a real and an imaginary part of an additional inductance component added to the antenna by received electromagnetic signals. The separating the signals from different physiological sources is in particular based on relative magnitudes of said detected real and imaginary inductance components added to the resonator circuit by the returned signals.

In one set of embodiments, a reference dataset is consulted which stores, for different signals from different known physiological sources within the body, an indicator of known characteristic relative magnitudes of real and imaginary inductance components added to a sensing antenna circuit by signals originating from that source. By consulting this dataset, and using the detected measures indicative of real and imaginary inductance components, one or more signal components from different known physiological sources are extracted from the received electromagnetic signals.

In other embodiments, different approaches may be used to extract the different physiological signals from the received composite signal based on the detected real and imaginary components of the added inductance component. For example, in one set of embodiments, an independent component analysis procedure can be applied to signals representative of the sensed measures indicative of real and imaginary parts of the additional inductance component added to the resonator circuit. This enables the different signal components contributing to the real and imaginary inductance signals, originating from different physiological sources, to be extracted. This will be explained in further detail below.

A number of example embodiments will be discussed below which are based on consulting a dataset storing information indicative of known characteristic relative magnitudes of real and imaginary added inductance components for different physiological sources. However, this represents only one example group of possible embodiments, and use of a dataset is not essential.

The invention provides a novel solution to the problem of separating out from a returned signal different signal components originating from different physiological sources, e.g. heart pulse, lung inflation changes, arterial behavior.

The invention is based on the insight that electromagnetic signals returned from the body from different physiological or kymographic origins exhibit different "fingerprints" in the form of relative strength of the real part and the imaginary part of a corresponding inductance component induced at the antenna by the returned signals (the reflected inductance). This insight provides a simple and efficient approach to separating signal components from different physiological sources.

One straightforward approach to applying or representing this mathematically is the use of vectors in the complex plane. The complete (composite) returned signal from the body can be represented as a 2D vector, with the real and imaginary parts of the additional induced inductance at the antenna (or measures related to or indicative thereof, such as antenna current frequency and amplitude) making up the vector components. Different known signal 'fingerprints' for different physiological sources can each be represented as 2D vectors, the vector components corresponding to relative magnitudes of real and imaginary parts of an inductance induced by a signal from that physiological source (or measures related to or indicative thereof). Detailed explanation of example embodiments will be described further below.

More broadly, signal components originating from different physiological or anatomical origins within the body can be understood as contributing added inductance components having different complex phase (different complex argument).

The general theory underlying embodiments of the present invention will now be described.

Embodiments of the invention provide an inductive sensing system and method, and are based therefore on principles of magnetic induction. The basic principles of magnetic induction will first be briefly outlined.

Embodiments of the invention operate on the principle of inductive coupling, whereby a coil or wire has induced across it a potential difference due to exposure to a time varying magnetic field. Embodiments of the present invention use this principle to measure strength of electromagnetic signals generated within regions of a body by sensing changes in the inductance of a coil or loop antenna placed in proximity to the body, where these changes are detected based on changing resonance characteristics of the antenna or resonator circuit.

Any electrical conductor exhibits a property of self-inductance. Self-inductance is the property of an electrical conductor by which a change in the current being driven through the conductor results in induction of an electromotive force in the conductor. According to Lenz' law, the induced electromotive force is in a direction such as to resist the change in current which is inducing it. It is hence commonly termed 'back-EMF'. Self-inductance can be understood as arising due to a magnetic flux induced as a result of the change in current (Ampere's law). This flux then interacts with the conductor itself to induce a back-EMF (Faraday's law of induction and Lenz' law).

The relationship between self-inductance L of a circuit, the voltage, v(t), and the current I(t) can be expressed as:

$$v(t) = L\frac{dI(t)}{dt} \tag{3}$$

By using Faraday's law of induction to express v(t) as $d\Phi_B/dt$ (where $\Phi_B$ is magnetic flux) and integrating with respect to time (assuming L to be time-constant), free-space self-inductance L for a coil of N turns can be expressed as:

$$L = N\Phi_B / I \tag{4}$$

Embodiments of the present invention make use of a resonator comprising an antenna (which may in preferred embodiments comprise only a single turn loop) to stimulate or excite a body with electromagnetic signals (waves) and to sense signals emitted back from the body in response to those excitation signals.

The coil may be driven with an alternating current to generate the excitation signals for application to the body. These may be propagating electromagnetic signals, propagated in to the medium in some cases, or the signals can consist of a non-propagating electromagnetic field applied to the medium, i.e. by bringing the loop antenna source into proximity of the target medium. The alternating current creates a field of alternating field strength.

When the coil is brought into proximity with a body, the inductance L acquires an additional reflected inductance component, $L_r$, arising due to eddy currents induced in the stimulated body as a result of application of the excitation signals.

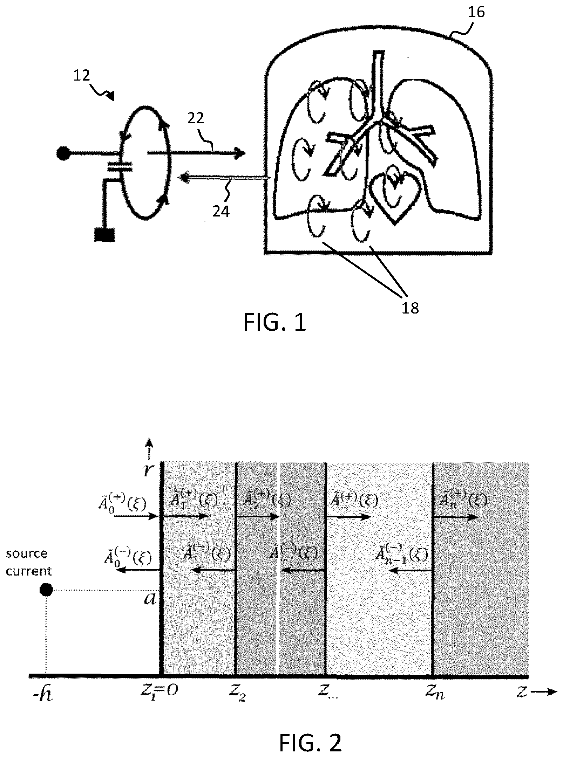

This is illustrated schematically in Fig. 1, which shows by way of example a loop antenna 12 being driven with an alternating current in proximity to a thorax 16 of a subject, so as to apply electromagnetic signals 22 to the thorax.

As a consequence, eddy currents 18 are induced within in the thorax.

These eddy currents in turn effectively make a contribution to the inductance of the loop antenna 12. This is because they themselves result in generation of a time-varying magnetic flux 24 of equivalent frequency to that generated by the primary antenna 12. These eddy-current fluxes combine with the primary flux of the antenna, resulting in a modified induced back-EMF in the antenna, and hence a larger measurable effective inductance.

The added component of inductance arising from the eddy currents is referred to as 'reflected inductance', $L_r$. The total inductance $L_t$ of the coil antenna 12 may be expressed as:

$$L_t = L_0 + L_r \tag{5}$$

where $L_0$ is the self-inductance of the coil antenna 12 in free space and $L_r$ is the reflected inductance caused by the presence of the proximate body.

Reflected inductance can be defined as:

$$L_r \equiv \frac{1}{I} \oint_{\text{all turns}} \boldsymbol{A}_r \cdot d\boldsymbol{l} \tag{6}$$

where $\boldsymbol{A}_r$ is the reflected part of the electromagnetic vector potential (i.e. the part that is generated by the eddy currents 18 in the stimulated medium), and $I$ is the coil current. The reflected inductance is closely related to the reflected impedance $Z_r$. The relationship is $L_r = Z_r / i\omega$, where $\omega$ is the radial frequency of the electromagnetic excitation signals 22 (the time-varying field applied to the body).

The above integral expression can be understood by applying the relationship:

$$\nabla \times \boldsymbol{A}_r = \boldsymbol{B}_r \tag{7}$$

where $\boldsymbol{B}_r$ is the 'reflected' magnetic field and then applying Stokes' theorem to re-express equation (6) as:

$$L_r \equiv \frac{N}{I} \iint_S \boldsymbol{B} \cdot d\boldsymbol{s} = \frac{N\Phi_B}{I} \tag{8}$$

where N = number of turns. It can be seen that this corresponds to the form of the simplified expression for inductance outlined in equation (4) above.

The magnitude of the reflected inductance component gives an indication of the strength of the 'reflected' electromagnetic signals emitted back from the body.

In general, the reflected inductance, $L_r$, is complex, and can be expressed as

$$L_r = L'_r + iL''_r \tag{9}$$

where $L'_r$ is related to a reactive impedance of the coil antenna and $L''_r$ is related to resistive impedance of the coil.

Complex $L_r$ can of course also be expressed in polar form:

$$L_r = \sqrt{|L'_r|^2 + |L''_r|^2}\, e^{i\varphi} \tag{10}$$

where $\varphi$ is the argument or phase of complex $L_r$ ( in general given by $\varphi = \arctan\left(\frac{|L''_r|}{|L'_r|}\right)$.

The addition of the reflected component of inductance $L_r$ leads to a detuning of electrical characteristics of the resonator circuit. In particular, both the natural radial frequency of the resonator circuit and the damping factor of the resonator circuit change. By measuring this detuning of the electrical characteristics, the real and imaginary parts of the reflected inductance $L_r$ can be detected.

In particular, the detuning of the characteristics of the coil as result of the addition of the reflected inductance can be expressed as follows:

$$\omega_{0,t} = \omega_{0,0} \sqrt{\frac{L_0}{L_0 + L'_r}} \tag{11}$$

$$\zeta_t = \zeta_0 \sqrt{\frac{L_0}{L_0 + L'_r}} + \frac{-L''_r}{2(L_0 + L'_r)} \tag{12}$$

where $\omega_{0,0} = \sqrt{\frac{1}{CL_0}}$ is the undamped natural radial frequency of the coil circuit in free space, $\omega_{0,t}$ is the natural undamped radial frequency of the coil circuit in the presence of a medium or body (the subscript t standing for 'total'), $\zeta_0 = \frac{\frac{R_0}{\omega_{0,0}}}{2L_0}$ is the damping factor in free space, $\zeta_t$ is the (total) damping factor in the presence of a medium, $L'_r$ is the real part of the reflected inductance defined in equation (9) and $L''_r$ is the imaginary part of the reflected inductance defined in equation (9).

It can be seen that the detuned natural radial frequency depends only on the real part of the reflected inductance $L_r'$. The detuned damping factor depends also upon the imaginary part of the reflected inductance $L_r''$.

For simplicity, it can be preferable to work with geometrically normalized quantities. Accordingly, a 'characteristic' self-inductance $\hat{L}_0$ and characteristic reflected inductance $\hat{L}_r$ can be defined as follows:

$$\hat{L}_0 \equiv \frac{L_0}{lN^2} \tag{13}$$

$$\hat{L}_r \equiv \frac{L_r}{lN^2} \tag{14}$$

where $l$ = circumference of a single turn of the coil, $N$ = number of coil windings, $L_0$ is free-space self-inductance (real), $L_r$ is reflected inductance (complex), and where $L_r$ is defined as in equation (6) above. The benefit of using the geometrically normalized quantities lies in the fact that $\hat{L}_0$ is independent of system size and the number of turns in the antenna coil.

Using these characteristic quantities, the detuning of the characteristics of the coil as a result of the addition of the reflected inductance can be expressed as follows:

$$\omega_{0,t} = \omega_{0,0}\sqrt{\frac{\hat{L}_0}{\hat{L}_0 + \hat{L}_r'}} \tag{15}$$

$$\zeta_t = \zeta_0\sqrt{\frac{\hat{L}_0}{\hat{L}_0 + \hat{L}_r'}} + \frac{-\hat{L}_r''}{2(\hat{L}_0 + \hat{L}_r')} \tag{16}$$

where $\omega_{0,0} = \sqrt{\frac{1}{CL_0}}$ is the undamped natural radial frequency of the coil circuit in free space, $\omega_{0,t}$ is the natural undamped radial frequency of the coil circuit in the presence of a medium or body (the subscript t standing for 'total'), $\zeta_0 = \frac{\frac{R_0}{\omega_{0,0}}}{2L_0}$ is the damping factor in free space, $\zeta_t$ is the (total) damping factor in the presence of a medium, $\hat{L}_r'$ is the real part of the characteristic reflected inductance defined in equation (9) and $\hat{L}_r''$ is the imaginary part of the characteristic reflected inductance defined in equation (9).

Above has been outlined the basic principles of magnetic induction upon which the sensing system of embodiments of the present invention relies.

As noted above, the invention is based on the insight that the relative magnitudes of real and imaginary parts of the additional reflected inductance component, $L_r$, vary in dependence upon the location within the body from which the reflected signal has originated. Another way of expressing this is that the complex phase or argument of $L_r$ varies depending upon the location within the body from which the corresponding reflected signal component originates. This principle allows signal components from different anatomical locations to be identified.

The theory behind this physical insight will now be explained in more detail. This will be done by showing how a spatial mapping can be built up showing the variation in phase or argument of a reflected inductance signal $L_r$ originating from different locations within a body.

In particular, it may be shown how to arrive at a spatial mapping $M(r', z')$ of originating sources of characteristic reflected inductance $\hat{L}_r$ (in units of nH/m), where total characteristic reflected inductance is given by:

$$\hat{L}_r = \iint_{\text{body}} dr' dz' M(r', z') \tag{16b}$$

The defined integral takes place over the depth coordinate (within the stimulated body), $z'$, and the transverse radial coordinate (within the stimulated body), $r'$. The spatial mapping function $M(r', z')$ represents a spatial mapping of the sources of sensed reflected inductance across the body.

First, a basic inductive sensing equation may be set out. This can be derived from Maxwell's equations, and is expressed using cylindrical coordinates (r, z, φ). Circular symmetry is assumed about the z-axis. Furthermore, the induced eddy currents in the body (which are to be taken as the source of the modeled 'reflected' or secondary fields) are taken to be confined purely to the radial plane, circulating in the azimuthal direction (i.e. the circumferential direction around the z-axis). The equation hence describes the purely azimuthal vector potential $A_\varphi(r, z)$ induced by a purely azimuthal current density source $J_{src, \varphi}$:

$$\left[\frac{1}{r}\frac{\partial}{\partial r}\left(r\frac{\partial}{\partial r}\right) + \frac{\partial^2}{\partial z^2} - \frac{1}{r^2} + k^2\right] A_\varphi = -\mu_0 J_{src,\varphi} \tag{17}$$

where $k^2 = \omega^2 \mu_0 \left(\varepsilon - \frac{i\sigma}{\omega}\right)$ is the spatially-varying total wavenumber squared, $\varepsilon(r, z)$ is the permittivity of the medium, $\sigma(r, z)$ is the conductivity of the medium, $\mu_0$ is the vacuum permeability, and $\omega$ is the radial frequency of the eddy currents. Due to the spatially dependent $\varepsilon(r, z)$ and $\sigma(r, z)$, the inductive sensing equation (17) allows for inhomogeneous media, and is thus not limited to stratified media. For the purpose of sensing biological tissues, the medium permeability, $\mu$, may be approximated as $\mu \approx \mu_0$.

In general, the solution of Maxwell's equations for a problem involving media will be identical to the solution of an equivalent free-space problem in which the source terms themselves include all of the induced currents and induced charges of the first problem. This permits construction of a solution using the free-space Green function of the inductive-sensing equation (17) and the total azimuthal current density:

$$A_\varphi(r, z) = \iint dr' dz' \; G_0(r, \; z; r', \; z') J_\varphi(r', z') \tag{18}$$

where $J_\varphi(r', z')$ is the total azimuthal current density, which comprises both the source and the induced currents, and $G_0(r, z; r', z')$ is the free-space Green function implicitly defined by

$$\left[\frac{1}{r}\frac{\partial}{\partial r}\left(r\frac{\partial}{\partial r}\right) + \frac{\partial^2}{\partial z^2} - \frac{1}{r^2} + k_0^2\right] G_0(r, \; z; r', \; z') = -\mu_0 \delta(r - r')\delta(z - z') \tag{19}$$

where $k_0^2 = \omega^2 \mu_0 \varepsilon_0$. The solution to the free-space Green function may be obtained by a Fourier transform in z, a subsequent a Hankel transform in $r$, and a subsequent inverse Fourier transform in $z$ using contour integration in the complex plane, yielding

$$G_0(r, \; z; r', \; z') = \mu_0 r' \int_0^\infty \frac{\mathcal{J}_1(\xi r)\mathcal{J}_1(\xi r')e^{-i\beta_0|z-z'|}\xi}{2i\beta_0} \; d\xi \tag{20}$$

where $\mathcal{J}_1(\xi r')$ is the first-order Bessel function of the first kind, $\beta_0 \equiv \sqrt{k_0^2 - \xi^2}$ is the complex longitudinal wavenumber in free space (chosen in the fourth quadrant), and $\xi$ is the transverse wavenumber (the wavenumber in the radial, r', direction).

The total current density, $J_\varphi$, in equation (18) comprises the source current in the antenna loop and the eddy currents in the probed body in linear summation, i.e.

$$J_\varphi(r, z) = J_{src,\varphi}(r, z) + J_{eddy,\varphi}(r, z) \tag{21}$$

This allows for straightforward separation of the reflected vector potential (the quantity which is sought) from the source vector potential in equation (18) to thereby arrive at

$$A_{\varphi,refl}(r, z) = \iint dr' dz' \; G_0(r, \; z; r', \; z') J_{eddy,\varphi}(r', z') \tag{22}$$

As discussed above, it is convenient to consider a 'characteristic' reflected inductance, $\hat{L}_r$ instead of the reflected inductance itself, meaning the reflected inductance, normalized by the antenna loop length and number of windings (see equation (14) above). Considering an antenna with a single loop winding, (i.e. N=1 in equation (14)), the characteristic reflected inductance may be derived from the reflected vector potential at the loop wire $A_{\varphi,refl}$ ($r = a, z = -h$), and using a combination of equations (14) and (6), as:

$$\hat{L}_r = \frac{A_{\varphi,\mathrm{refl}}(r=a,z=-h)}{I} \tag{23}$$

where $I$ is the loop current, $a$ is the loop radius, and $h$ is the distance between the loop and the probed medium (assuming the surface of the probed medium to be at z=0 and the medium extending in the region where z>0).

Combining equations (22) and (23) yields an equation describing the (sought) spatial mapping of the characteristic reflected inductance signal, as a function of location (r, z) of the source of the reflected signal within the probed body:

$$\hat{L}_r = \iint dr'dz' \ G_0\left(a, \ -h; r', \ z'\right)\frac{J_{\mathrm{eddy},\varphi}\left(r',z'\right)}{I} \tag{24}$$

where the free-space Green function, $G_0$, is given by equation (20).

The right-hand side of equation (24) corresponds to the right-hand side of the spatial mapping equation (16b) introduced above, namely: $\hat{L}_r = \iint_{\mathrm{body}} dr'dz' \ M(r', z')$. Hence, the spatial mapping function, M(r', z') can be represented as follows:

$$M(r', z') = G_0(a, \ -h; r', \ z')\frac{J_{\mathrm{eddy},\varphi}(r',z')}{I} \tag{24b}$$

This spatial mapping, M, is the spatial distribution of originating sources of the characteristic reflected inductance $\hat{L}_r$ within the stimulated body.

Equation (24) (and equation (18)) describe the expected magnitude of the additional component of inductance (the reflected inductance component) induced in the resonator circuit by a returned (reflected) electromagnetic signal originating from a location in the probed body (r', z') (where circular symmetry about the azimuthal direction, $\varphi$, is assumed).

It can be seen from equation (24) therefore that the characteristic reflected inductance, $\hat{L}_r$, which may be understood as the quantification of a returned (reflected) electromagnetic signal from the body, may be expressed as a convolution of a "loop-sensitivity-kernel", $G_0(a, -h; r', z')$, over the eddy-currents, normalized to the loop current $\frac{J_{\mathrm{eddy},\varphi}(r',z')}{I}$.

The loop sensitivity kernel, $G_0$, is a function of position (r', z') within the stimulated body, and effectively quantifies the effect of an eddy current at position (r', z') normalized to the loop current, on the characteristic reflected inductance sensed at the resonator circuit. This is represented in mathematical form in equation (24) above. A simple multiplication of the loop sensitivity kernel with the normalized eddy-currents thus provides the spatial mapping, M (r', z'), of the origin of the reflected inductance within the stimulated body, as shown in equation (24b). The spatial mapping M (r', z') can be directly interpreted as the spatial distribution of the origin of the characteristic reflected inductance, $\hat{L}_r$, within the body.

In order to quantitatively model or map the reflected inductance as a function of position (as described in equation (24)), it is necessary to compute the induced eddy currents $J_{\mathrm{eddy},\varphi}(r, z)$. The eddy currents comprise both conduction currents and polarization currents, i.e.

$$J_{\mathrm{eddy},\varphi}(r,z) = \left(-i\omega\sigma(r,z) + \omega^2\varepsilon_0\chi_e(r,z)\right)A_\varphi(r,z) \tag{25}$$

where $\chi_e = \frac{\varepsilon}{\varepsilon_0} - 1$ is the electric susceptibility, $\varepsilon$ is the permittivity of the medium, $\sigma(r, z)$ is the is the conductivity of the medium, and $\omega$ is the eddy current radial frequency.

The spatial mapping, M (r', z'), of the characteristic reflected inductance sources may now be found by combining equations (25) and (24b), yielding:

$$M(r', z') = G_0(a, \ -h; r', \ z')[-i\omega\sigma(r',z') + \omega^2\varepsilon_0\chi_e(r',z')]\frac{A_\varphi(r',z')}{I} \tag{25b}$$

The mapping function, M(r, z'), consists of three factors. The factor $\frac{A_\varphi(r',z')}{I}$ is the vector potential normalized to the loop current. This last factor multiplied with the factor [...] in the middle yields the eddy current density normalized to the loop current. These two factors multiplied by the loop-sensitivity kernel $G_0(a, -h; r', z')$ then yields the spatial mapping $M(r', z')$. The characteristic reflected inductance, $\hat{L}_r$, may then be obtained by simply integrating with respect to r' and z' (as shown in equation (16b) above).

It can be immediately seen from equation (25b) above that the spatial mapping M (r', z') has real and imaginary components which are each differently dependent upon position of the reflected inductance source (r', z') within the probed body. Hence the relative magnitudes of the real and imaginary components of M(r', z') for a given $\hat{L}_r$ component depend upon the location of the eddy current source within the body giving rise to that component. Since characteristic reflected inductance, $\hat{L}_r$, is directly dependent on M (r', z') via equation (16b) above, it can be directly seen that the relative magnitudes of the real and imaginary components of $\hat{L}_r$ for a given eddy current source depend upon the location of that source within the body.

This relation allows returned signal components originating from different physiological sources within the body to be distinguished based on their different characteristic relative magnitudes of real and imaginary components of $\hat{L}_r$.

As indicated by equation (25b), a final quantified solution to $M(r', z')$ requires a solution to $A_\varphi(r, z)$ (equation (17)) itself. A solution to $A_\varphi(r, z)$ may be determined in at least two different ways.

A first way is more generic, and follows a general perturbative approach. By combining equations (25), (21) and (18), a solution to equation (17) can be determined as a perturbation series:

$$A_\varphi(r,z) = \iint dr'dz' \ G_0(r, z; r', z')J_{\text{src},\varphi}(r',z') \tag{26}$$
$$+ \iint dr''dz'' \iint dr'dz' \ G_0(r, z; r'', z'')\mathcal{V}(r'',z'')G_0(r'', z''; r', z')J_{\text{src},\varphi}(r',z')$$
$$+ \dots$$

where $\mathcal{V}(r, z) \equiv -i\omega\sigma(r, z) + \omega^2\varepsilon_0\chi_e(r, z)$ is the local perturbation of the series. This solution is generic as it applies to any circular symmetric geometry. However, it is in general relatively computationally intensive to obtain the final result.

A second way of determining a solution to $A_\varphi(r, z)$ will now be described. This approach is computationally more efficient, but can be applied to layered media only (i.e. media comprising multiple stacked layers each comprised of a medium of a different density, where the density is assumed to be homogeneous within each individual layer). A first step is to decompose the solution into a right-traveling (+) and left-traveling (-) part:

$$A_\varphi(r,z) = A_\varphi^{(+)}(r,z) + A_\varphi^{(-)}(r,z) \tag{27}$$

In each individual homogeneous layer, the right-traveling and left-traveling parts may be further decomposed into (r, z)-separated modes of the homogeneous inductive sensing equation (17). These modes are parameterized by a real wavenumber $\xi$ in the radial (or transverse), r, direction, yielding

$$A_\varphi^{(+)}(r,z;\xi) = \mathcal{J}_1(\xi r)e^{-i\beta z} \tag{28}$$

$$A_\varphi^{(-)}(r,z;\xi) = \mathcal{J}_1(\xi r)e^{+i\beta z} \tag{29}$$

where $\mathcal{J}_1(\xi r)$ is the first-order Bessel function of the first kind, and $\beta \equiv \sqrt{k^2 - \xi^2}$ is the longitudinal wavenumber (the wavenumber in the z-direction) chosen in the fourth quadrant.

An amplitude profile $\tilde{A}_\varphi^{(\pm)}(\xi, z)$ can be uniquely determined by a Hankel transform

$$\tilde{A}_\varphi^{(\pm)}(\xi,z) = \int_0^\infty dr \ A_\varphi^{(\pm)}(r, z)\mathcal{J}_1(\xi r)r \tag{30}$$

$$A_\varphi^{(\pm)}(r,z) = \int_0^\infty d\xi \ \tilde{A}_\varphi^{(\pm)}(\xi, z)\mathcal{J}_1(\xi r)\xi \tag{31}$$

The amplitude profile $\tilde{A}_\varphi(\xi, z)$ may be expressed in terms of the (r,z)-separated modes, as follows:

$$\tilde{A}_\varphi(\xi, z) = \begin{cases} \tilde{A}_{0,\varphi}^{(+)}(\xi)e^{-i\beta_0|z|} + \tilde{A}_{0,\varphi}^{(-)}(\xi)e^{i\beta_0|z|} & , z < 0 \\ \tilde{A}_{1,\varphi}^{(+)}(\xi)e^{-i\beta_1|z-z_1|} + \tilde{A}_{1,\varphi}^{(-)}(\xi)e^{-i\beta_1|z-z_2|} & , 0 < z < z_2 \\ \dots & \dots \\ \tilde{A}_{n-1,\varphi}^{(+)}(\xi)e^{-i\beta_{n-1}|z-z_{n-1}|} + \tilde{A}_{n-1,\varphi}^{(-)}(\xi)e^{-i\beta_{n-1}|z-z_n|} & , z_{n-1} < z < z_n \\ \tilde{A}_{n,\varphi}^{(+)}(\xi)e^{-i\beta_n|z-z_{n-1}|} & , z > z_n \end{cases} \qquad (32)$$

where $\tilde{A}_{x,\varphi}^{(\pm)}(\xi)$ are the Hankel-transformed amplitude profiles of the right and left-traveling fields on the layer interfaces, as shown in Fig. 2, $\beta_x \equiv \sqrt{k^2 - \xi^2}$ is the longitudinal wavenumber in layer number $x$, and $Z_1, Z_2,...Z_n$ corresponding to depths in the z-direction of layer numbers 1, 2,...$n$.

The multiple stacked layers, and corresponding right and left-traveling fields on the layer interfaces are illustrated in Fig. 2. The x-axis corresponds to distance in the z-direction, with $Z_1, Z_{2,...}Z_n$ corresponding to depths in the z-direction of the layers 1, 2,...n.

The incoming amplitude profile may be determined from equations (18) and (20), and using a source current density:

$$J_{\text{src},\varphi}(r,z) = I\delta(r-a)\delta(z+h) \qquad (33)$$

where $I$ is the antenna current, $a$ is the antenna loop radius, and $h$ is the distance between the antenna loop and the probed medium (assuming the surface of the probed medium to be at z=0 and the medium extending in the region where z>0). This yields:

$$\tilde{A}_{o,\varphi}^{(+)}(\xi) = \mu_0 I a \frac{J_1(\xi a)e^{-i\beta_0 h}}{2i\beta_0} \qquad (34)$$

Using transfer matrix theory (well known in the present field for instance for modelling optical waves in multilayer media) with continuous $A_\varphi(r, z)$ and continuous $\frac{\partial}{\partial z}A_\varphi(r, z)$, is possible to express all the other coefficients in equation (32) above and Fig. 2 in terms of $\tilde{A}_{o,\varphi}^{(+)}(\xi)$ known from equation (34).

Since these expressions are quite lengthy, for brevity, the full set of expressions is omitted for the present purposes. However, it can be seen that there is a second way to compute $A_\varphi(r, z)$ that works for stratified media, and is computationally more efficient than the first perturbative approach of equation (26). This is based on calculating the total Hankel-transformed vector potential $\tilde{A}_\varphi(\xi, z)$ of equation (32) using equation (34) and applying transfer matrix theory. The vector potential in real space $A_\varphi(r', z')$ is also computed via an inverse Hankel transform defined by equation (31) and equation (27), thereby enabling (via equation (25b)) the spatial mapping $M(r', z')$ of the characteristic reflected inductance $\hat{L}_r$ to be expressed more explicitly as follows:

$$M(r', z') = G_0(a, -h; r', z')[-i\omega\sigma(r',z') + \omega^2\varepsilon_0\chi_e(r',z')]\int_0^\infty d\xi \frac{\tilde{A}_\varphi(\xi,z')}{I}J_1(\xi r')\xi$$

where $\frac{\tilde{A}_\varphi(\xi,z')}{I}$ is the total Hankel-transformed vector potential computed from equation (32), normalized to the loop current.

As discussed above, it can be seen that the relative magnitudes of the real and imaginary components of M (r', z') (and hence its complex argument or phase) depend upon the position, (r', z'), of the $L_r$ signal sources within the probed body. Accordingly, the same is true for the reflected inductance itself which directly depends upon M (r', z'). Hence, relative magnitudes of real and imaginary components of $\hat{L}_r$ for a given physiological signal source depends upon the location of the source within the probed body.

Using the derived solution for $A_\varphi(r, z)$ and therefore for $J_{\text{eddy},\varphi}(r, z)$ (equation (25)), it is possible to derive a spatial mapping of the expected amplitude and phase of an induced reflected inductance component, $L_r$, at the antenna 12 for signals as a function of originating location of the signal within the probed body.

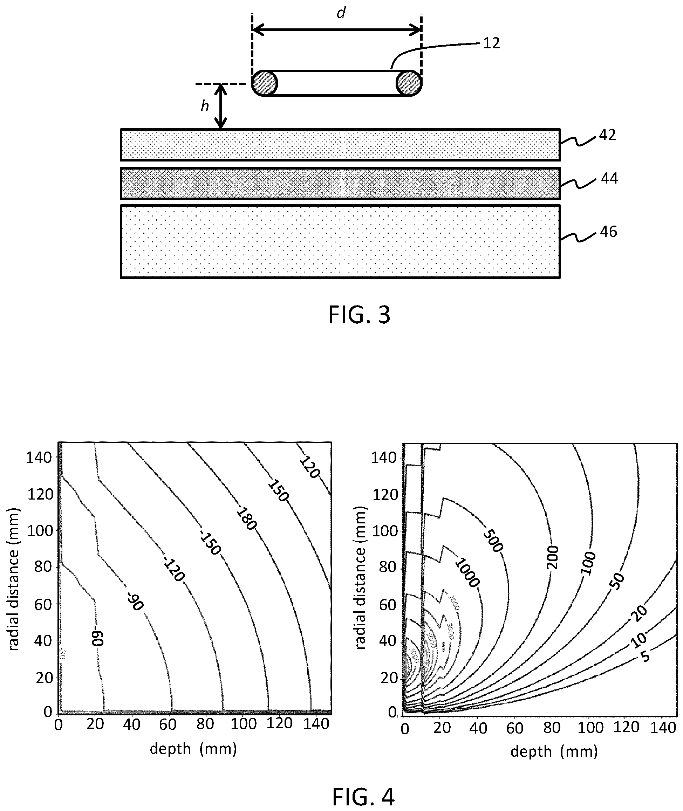

An example spatial mapping was derived for an example system having the arrangement illustrated in Fig. 3. An example antenna 12 having a single loop winding of radius *d*, was modeled with a separation distance, *h*, from the body being probed. The probed body is modeled as comprising three layers, 42, 44, 46, of different medium conductivity σ and permittivity ε. Layer 42 is modeled as a fat layer of depth 10 mm, layer 44 as a bone cancellous layer of depth 10 mm, and further layer representative of an inflated lung. The antenna height *h* was modeled at 5 mm, and the antenna diameter was modeled at 50 mm. The frequency, f, of generated electromagnetic excitation signals was modeled as *f* = 200 MHz. The corresponding normalized radial frequency ω^ = ω / 2πc, where c is the speed of light and ω = 2πf is the radial frequency is approximately ω^ = 0.1.

Fig. 4 shows a contour plot illustrating the phase and amplitude of characteristic reflected inductance $\hat{L}_r$ (as represented in equation (24)) for the setup of Fig. 3, as a function of depth (z-direction) and radial position (r direction) within the medium. The left-hand plot shows the phase (argument) of (complex) $\hat{L}_r$. The right-hand plot shows the magnitude of $\hat{L}_r$. The x-axis corresponds to depths in the z-direction within the medium (units: mm). The y-axis corresponds to radial position (along the r-direction) (units: mm).

It can be seen that the phase of the expected characteristic reflected inductance $\hat{L}_r$ varies as a function both of depth, z, and radial position, r within the medium. As noted above, reference to characteristic reflected inductance $\hat{L}_r$ is merely for convenience, with $\hat{L}_r$ related to non-normalized reflected inductance, $L_r$, by $\hat{L}_r \equiv \frac{L_r}{lN^2}$, where *l* is the length of the antenna loop windings and N is the number of antenna windings. It can be seen that the phase therefore also varies as a function of position within the medium for $L_r$.

The above outlined theory, and the plot in Fig. 3 demonstrates that the complex argument or phase of the reflected inductance, $L_r$, (or characteristic reflected inductance) varies in dependence upon position of the source of the returned electromagnetic signal in the body, and that therefore the relative magnitudes of real and imaginary parts of the detected reflected inductance also vary in dependence upon position of the source of the returned electromagnetic signal in the body (see equation 10).

It thus follows that the relative magnitudes of real and imaginary parts of the detected reflected inductance can be used to distinguish and separate returned signals from different physiological sources. Embodiments of the invention are based on this principle.

One example inductive sensing system 8 in accordance with one or more embodiments is shown in schematic form in Fig. 3. This example system will now be outlined.

The inductive sensing system 8 is for sensing electromagnetic signals returned from a body responsive to application of electromagnetic excitation signals into said body.

The system comprises a resonator circuit 10 comprising a loop antenna 12 and an electrically coupled capacitor 13. The capacitance of the capacitor 13 at least partially defines a natural resonance frequency of the resonator circuit (in the absence of forcing or damping). When the antenna 12 is excited, it will tend to naturally resonate at the defined resonance frequency, generating electromagnetic signals at the same frequency. Selecting the capacitance of the capacitor hence allows for at least partial tuning of the frequency of the generated electromagnetic signals.

The system 8 further comprises a signal generation means 14 adapted to excite the loop antenna to generate the electromagnetic excitation signals. The signal generation means may comprise a driver means for driving the antenna, for instance at a radial frequency *ω*, i.e driving the antenna with an alternating current of frequency ω. The driving means may be or comprise an oscillator for instance.

The signal generation means may drive the antenna and resonator circuit with a current of radial frequency ω where excitation signals of radial frequency ω are required.

By exciting the resonator, a resonating current is induced to flow back and forth through the loop antenna into the capacitor. By driving an alternating current through the antenna generation of oscillatory electromagnetic signals (waves) may thereby be stimulated.

The same antenna is used to generate the excitation signals as is used to sense the electromagnetic signals received from the body in response.

For the avoidance of doubt 'electromagnetic excitation signals' simply means electromagnetic signals for applying to the body for the purpose of exciting or stimulating generation of eddy currents within the body for in turn stimulating emission of electromagnetic signals back out of the body which can be sensed by the sensing system.

By 'electromagnetic signals' may generally be meant electromagnetic radiation emissions or electromagnetic near-field oscillations or electromagnetic oscillations and/or electromagnetic waves.

The system 8 further comprises a signal sensing means ("Signal Sense") 20 adapted to sense said returned signals from the body using the loop antenna 12, based on detecting variations in electrical characteristics of the resonator circuit 10. The signal sensing means may include signal processing or analysis means for detecting or monitoring electrical characteristics of the current in the resonator circuit 10.

For example, the signal sensing means 20 may be adapted to monitor at least a frequency of the resonator circuit current, and an amplitude of the resonator circuit current. These properties of the current will change in dependence upon the strength of the reflected electromagnetic signals returned from the body and detected at the antenna. The changes in these properties are indicative of the magnitudes of the real and imaginary parts respectively of the additional inductance component induced at the resonator circuit (the reflected inductance $L_r$) by the received returned electromagnetic signals.

Sensing of these signal characteristics is performed at the same time (i.e. simultaneously with) excitation of the antenna for generating the excitation signals. Hence signal transmission and sensing is performed simultaneously.

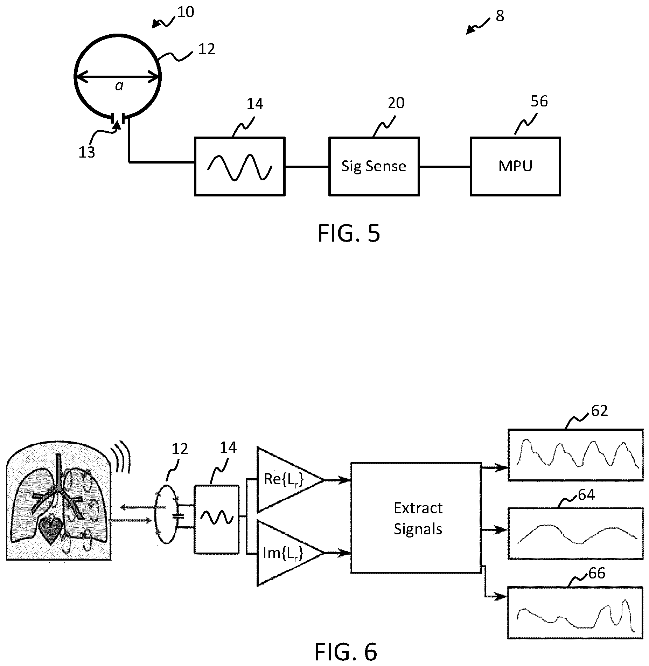

In Fig. 5, the signal sensing means 20 is shown connected to the resonator circuit 10 via the signal generation means 14. However, this is not essential: the signal sensing means and signal processing means may be independently connected to the resonator.

The system 8 is adapted to perform a series of steps using the above components. The system may include a controller or microprocessor ("MPU") 56 adapted to perform or facilitate these steps. An example microprocessor 56 is shown in the example system of Fig. 5 for illustration. However, a dedicated controller or microprocessor is not essential. In other examples, one or more of the other components of the system, such as the signal sensing means 20 and/or signal generation means 14 may be adapted to perform the steps.

The system 8 is adapted to perform the following steps:

detect from the sensed returned signals measures indicative of both a real and an imaginary part of an additional inductance component induced in the resonator circuit 10 by the received electromagnetic signals, and
using the detected measures indicative of real and imaginary inductance components, extract from the returned electromagnetic signals one or more individual signal components corresponding to different respective physiological sources, the extraction being based on relative magnitudes of said detected real and imaginary inductance components added to the resonator circuit 10 by the returned signals.

In some embodiments, the system is adapted to access a dataset which stores, for signals from a plurality of different known physiological sources within said body, information indicative of characteristic relative magnitudes of real and imaginary inductance components added to a resonator circuit by a signal received from said physiological source; and

using the detected measures indicative of real and imaginary inductance components, and based on consulting said dataset, extract from the returned electromagnetic signals the one or more individual signal components corresponding to different respective known physiological sources.

In other embodiments a different means for separating the different physiological signals may be used, for example an independent component analysis method.

The additional inductance component mentioned above, may, for brevity, be referred to as the reflected inductance, $L_r$, and corresponds to the reflected inductance discussed in detail above. Complex $L_r$ can be represented as $L_r = L'_r + iL''_r$, with $L'_r$ being the real part, and $L''_r$ being the imaginary part.

The sensed measures indicative of real and imaginary parts of the additional inductance component may take different forms. In an advantageous set of examples however, the sensed measure indicative of the real part may be sensed changes in the frequency of the resonator circuit 10 current. The sensed measure indicative of the imaginary part of $L_r$ may be sensed changes in the amplitude of the resonator circuit current.

Table 1 below sets out the various electrical characteristics which change or vary in dependence upon changes in the real and imaginary parts of the reflected inductance at the antenna. Each therefore provides a potential measure indicative of one of the real or imaginary parts of the reflected inductance $L_r$. Different characteristics are measurable in different of the system 8 components.

**Table 1**

| Received Inductive-sensing signal | Loop antenna | Loop resonator (= loop antenna + capacitor) | Oscillator (= loop resonator + oscillator electronics) |
|---|---|---|---|
| Real part $\mathrm{Re}[L_r] \equiv L'_r$ | Leads to a modified antenna reactance $\omega L'_r$ | Leads to a modified resonator frequency | Leads to a modified oscillator frequency |
| Imaginary part $\mathrm{Im}[L_r] \equiv L''_r$ | Leads to a modified antenna resistance $-\omega L''_r$ | Leads to a modified resonator damping | Leads to a modified oscillator amplitude |

Hence, any measure indicating changes in reactance and resistance respectively of the loop antenna may be used as measures indicative of the real and imaginary parts of the reflected inductance. A convenient direct measure is that of changes in frequency and damping of the resonator circuit.

The change in damping of the resonator circuit leads to a change in current amplitude of the resonator circuit. Hence, the imaginary part of $L_r$ can be measured by measuring changes in the resonator circuit current amplitude. It might also be measured via measuring changes in the voltage amplitude of the resonator circuit, where the relation between current amplitude, $I_0$, and voltage amplitude, $V_0$, is given by $I_0 = 2\pi CV_0$, where C is the total capacitance of the resonator circuit.

These can be sensed in turn for instance by sensing changes in the frequency and amplitude of the current in the resonator circuit or in the whole circuit including the signal generation means (e.g. the oscillator according to the example above).

As noted above, in accordance with at least one group of embodiments, the system is adapted to consult a dataset storing information indicative of characteristic relative magnitudes of real and imaginary parts of a reflected inductance component $L_r$ detected at the antenna for signals originating from different known physiological sources.

The dataset may be stored locally, for instance in a memory further comprised by the system, or the dataset may be stored externally, e.g. remotely from the system 8, e.g. in a remote computer or server or data store. The system may be adapted to communicate with such a remote computer or data store to thereby access the information stored therein.

The dataset may take the form for instance of a database, or a table, or any other form of data structure.

In accordance with one advantageous set of examples, the indicative information stored for each physiological source may take the form of a vector, having vector elements whose contents are indicative of said characteristic relative magnitudes of real and imaginary parts of the reflected inductance $L_r$.

These stored characteristic vectors for the different physiological sources, *X*, may hence take the form

$$\vec{P}_X = \begin{pmatrix} P_{X,\mathrm{re}} \\ P_{X,\mathrm{im}} \end{pmatrix} \tag{35}$$

where $P_{X,re}$ and $P_{X,im}$ are indicative of the relative magnitudes of the real and imaginary components of the reflected inductance signal expected to be induced by a signal originating from physiological source X.

These vectors may be referred to as 'fingerprint vectors', since they indicate an inductance signal fingerprint of each physiological source.

Examples of different physiological sources include, by way of non-limiting example, "breathing", "pulse", "motion" or "radial artery vessel diameter". The sources may include even more specific sources such as, "talking" or "eating".

The characteristic fingerprint vectors may be determined in advance and statically stored in the database, or may be dynamically updated during use of the system. The latter option will be described in more detail in passages to follow.

Initially deriving the fingerprint vectors may be performed empirically, or may be performed based on modeling for example. Empirical derivation may be based on an iterative sampling process, trialing different possible vectors, and iteratively moving toward a vector for each physiological source which accurately results in an extraction of a signal component corresponding to the physiological signal.

In a simplest case, the characteristic fingerprint vectors $\vec{P}_X$ may take the form of a normalized vector in the complex plane representative of the expected induced reflected inductance, $L_{r\_X}$, at the antenna by a signal originating from physiological source X. In this case

$$P_{X,\mathrm{re}} = \frac{Re\{P_X\}}{\sqrt{(Re\{P_X\})^2 + (Im\{P_X\})^2}} \tag{36}$$

$$P_{X,\mathrm{im}} = \frac{Im\{P_X\}}{\sqrt{(Re\{P_X\})^2 + (Im\{P_X\})^2}} \tag{37}$$

In this example, the characteristic vector $\vec{P}_X$ represents just the relative magnitudes of the real and imaginary components. It may be understood as effectively representing a direction of the expected complex $L_r$ in the complex plane.

The system 8 is further adapted to extract from the sensed electromagnetic signals one or more signal components from different known physiological sources using the stored information (i.e. the characteristic vectors in this example).

The full composite returned signals may be represented also in vector form, in terms of the real and imaginary parts of the additional inductance component (the reflected inductance) sensed at the antenna. The sensed signal, $\vec{S}(t)$ may hence take the form

$$\vec{S}(t) \equiv \begin{pmatrix} L_r'(t) \\ L_r''(t) \end{pmatrix} \tag{38}$$

where the detected additional inductance component, $L_r$, is given by $L_r(t) = L_r'(t) + iL_r''(t)$.

Extraction of a signal component $A_X(t)$ from a particular physiological source, X, may then comprise a step of projecting the sensed signal $\vec{S}(t)$ onto the stored characteristic vector $\vec{P}_X$ for that source. Mathematically, this may be achieved by performing a simple inner product of the sensed signal $\vec{S}(t)$ with the characteristic vector $\vec{P}_X$ for the given physiological source:

$$A_X(t) = \vec{P}_X \cdot \vec{S}(t) \tag{39}$$

This will result in a component of the inductance signal, $A_X(t)$, which corresponds only to the electromagnetic signals received from the particular physiological source X, having relative $L_r$ real and imaginary parts represented by $\vec{P}_X$.

This projection can be done for any one or more of the physiological sources having characteristic vectors stored in the dataset.

Optionally, the projection in equation (39) may be improved by first removing the baseline of $\vec{S}(t)$, e.g., by applying a high-pass filter on $\vec{S}(t)$ before taking the inner product of equation (39):

$$A_X(t) = \vec{P}_X \cdot \left( \vec{S}(t) - \vec{S}_{lp}(t) \right) \tag{39b}$$

where $\vec{S}_{lp}(t)$ is the slowly varying baseline component of $\vec{S}(t)$. Note that $\vec{S}_{lp}(t)$ is just a low-pass-filtered version of $\vec{S}(t)$). The 3-dB point of this filter should preferably be sufficiently low to ensure that relevant physiological changes are still present in the preprocessed signal $\vec{S}(t) - \vec{S}_{lp}(t)$.

Optionally the projection in equation (39) can be further improved by applying a band-pass filter before taking the inner-product:

$$A_X(t) = \vec{P}_X \cdot \left( \vec{S}(t) - \vec{S}_{lp}(t) - \vec{S}_{hp}(t) \right) \tag{39c}$$

where $\vec{S}_{hp}(t)$ is a high-pass-filtered version of $\vec{S}(t)$). The 3-dB point of this filter should preferably be sufficiently high to ensure that relevant physiological information is still present in the preprocessed signal $\vec{S}(t) - \vec{S}_{lp}(t) - \vec{S}_{hp}(t)$.

According to one or more sets of embodiments, the system 8 may include means for quantifying the physiological parameter or phenomenon to which one or more of the extracted signal components correspond. For instance, an extracted $L_r$ signal component originating from the lungs may correspond to the physiological parameter of lung volume. The system may include an algorithm or operator or function for converting or transforming or mapping the corresponding signal component into a signal representative of the corresponding physiological phenomenon e.g. lung volume.

In some cases, this may comprise a simple multiplier which is applied to the derived (raw) signal component for instance. In further cases, this may comprise a more complex function or operator which processes or transforms the raw derived signal component into a signal representative of the physical phenomenon or parameters.

The algorithms, operators and/or functions for performing this transformation or mapping to the physiological signals may be pre-stored locally or remotely and accessed by the system 8.

In some examples, mapping to the true physiological signals may be built in to the characteristic (fingerprint) vectors $\vec{P}_X$. For instance, instead of using simple normalized $L_r$ vectors having vector components of the form represented in equations (36) and (37) above, according to one or more examples, the components of the $\vec{P}_X$ vectors may be given by the magnitude of the true physiological parameter or phenomenon X itself, divided by the real and imaginary parts of the corresponding reflected inductance.

For example, if physiological phenomenon X is lung volume variation, then the corresponding fingerprint vector components $P_{X,re}$ and $P_{X,im}$ would have units of liters / Henrys. This then means that when an inner product is taken between the fingerprint vector and the sensed $L_r$ vector (units Henrys), the resulting output signal has units simply of liters (i.e. the true physiological parameter units).

A schematic representation of the processing steps performed by the system 8 in extracting the different signal components is illustrated in Fig. 6. Fig. 6 shows the loop antenna 12 and the coupled signal processing means 14. Generated excitation signals are shown transmitted, by way of example, to the thorax region of a subject body.

Returned electromagnetic signals from the body are received at the same antenna. An additional inductance component, $L_r$, is induced at the resonator circuit due to the received electromagnetic signals.

The real and imaginary parts of $L_r$ are detected and determined, and these are output as two independent signals.

As noted above, according to one set of examples, the real part of $L_r$ may be measured by measuring the frequency of the resonator circuit (comprising the antenna 12 and the coupled capacitor), or the frequency of the signal generation means 14 (e.g. free running oscillator). This is because the radial frequency of the resonator depends on the real part of the reflected inductance via the relationship

$$\omega_{0,t} = \omega_{0,0} \sqrt{\frac{L_0}{L_0 + \text{Re}[L_r]}} \qquad (40)$$

where $L_0$ is the characteristic self-inductance in free space, and $\omega_{0,0}$ is the radial frequency of the resonator in free space.

In other examples, the real part of $L_r$ may be measured in a different way, wherein the frequency of the resonator circuit and/or oscillator is held fixed through a control circuit, and wherein the real part of $L_r$ is measured via a frequency feedback signal to the control circuit. Such a circuitry is often known as a frequency-locked-loop circuit.

For example, a control circuitry may be implemented for maintaining the frequency of the resonator circuit at a fixed frequency. For this, frequency of the circuit may be measured recurrently on an ongoing basis and this frequency fed back to a variable capacitance capacitor component (as a feedback signal). This variable capacitor may adjust the capacitance of the resonator circuit in dependence upon the measured frequency so as to compensate for any sensed frequency changes, and maintain the frequency at a defined value.

The frequency feedback signal to the variable capacitor may then be used for determining the real part of $L_r$, since it contains information representative of changes in the natural frequency of the resonator (and oscillator) circuits.

Frequency-locked loop circuits such as these have advantages for instance for enabling regulatory compliance, since the emission frequency is held fixed, and cannot for example move into legally forbidden electromagnetic bands.

As also noted above, the imaginary part of $L_r$ may, according one set of examples, be measured by measuring the damping of the resonator. The damping of the resonator may in examples be measured by measuring the amplitude of the signal generation means (the oscillator 14 in this example). It may also be measured by measuring the voltage amplitude of the resonator circuit over the capacitor (often referred to as "tank swing" in the art), or the current amplitude of the resonator circuit.

This is because the oscillator 14 amplitude depends at least partly upon the imaginary part of the reflected inductance, due to an additional induced resistance component, $R_r$, in the resonator circuit having the magnitude

$$R_r = -\omega \text{Im}[L_r] \qquad (41)$$

where $\omega$ is the angular frequency of the resonator circuit current. This additional resistance component causes a reduction in the oscillator 14 amplitude, with the amplitude decreasing with increasing resistance.

According to certain examples, the damping of the resonator may be measured by using a fixed current and voltage amplitude control loop circuit, operating on similar principles to the fixed frequency feedback circuit discussed above for measuring the real part of $L_r$.

In particular the resonator circuit may be coupled to a gain controller or a damping controller for controlling (setting) a gain or a damping of the resonator circuit. During operation, the current or voltage amplitude of the resonator circuit may be monitored and this fed back to the gain controller via a feedback signal. The gain or damping controller may be adapted to adjust the gain or damping of the resonator circuit based on the returned feedback signal so as to compensate for any sensed changes in current or voltage amplitude and maintain the current amplitude or voltage amplitude at a defined fixed level. This defined level may be adjustable (e.g. user adjustable) according to preferred examples.

The current or voltage amplitude feedback signal fed to the gain or damping controller may then be used for measuring the imaginary part of $L_r$ since it contains information representative of instantaneous current or voltage amplitude of the resonator circuit.

The gain may be controlled based on controlling a tail-current of the resonator circuit (or the oscillator circuit) or a collector voltage (i.e. Vcc) of the gain circuit. The damping of the resonator circuit may be adjusted based on use of a variable resistor component in the resonator circuit.

Again, an advantage of using a feedback loop is enabling easier compliance with regulatory standards, e.g. requirements for circuits to operate within certain defined voltage or current amplitude limits.

Signals from different physiological origins are extracted by the system 8 based on any of the procedures and approaches outlined above.

As discussed, one approach is to use vector representation, and wherein the system accesses stored characteristic (or fingerprint) vectors $\vec{P}$ for different physiological sources, representing characteristic relative magnitudes of real and imaginary $L_r$ components for signals originating from the respective sources.

According to one set of examples, the fingerprint vectors $\vec{P}$ for different physiological sources may be pre-stored, for instance in a local memory, or remotely at a remote computer or server.

In some examples, the system may extract signal components for all of the physiological sources for which there are fingerprint vectors stored in the dataset i.e. the system may apply all of the fingerprint vectors stored in the dataset to the composite $L_r$ signal sensed by the system 8.

According to other examples, the system may include a user interface, and the system may be adapted to permit selection by a user of a set of one or more of the physiological sources for which there are fingerprint vectors stored in the dataset.

According to one or more examples, the system may be adapted to receive user input indicative of one or more patient demographic parameters, e.g. weight, age, height), and wherein the system is adapted to determine from the input patient parameters one or more relevant physiological sources to derive signal components for. The relevant fingerprint vectors corresponding to the determined physiological sources may then be retrieved from the dataset and used by the system 8 to extract the corresponding signal components.

In some cases more than one vector may be stored in the dataset for a given physiological source or one or more of the stored vectors may include patient dependent parameters which may be adjusted. A user interface may be used to receive user input indicative of one or more of these patient parameters, and these used to tune or adjust the vectors, or to select a most relevant fingerprint vector to apply in a given scenario. This option is based on the realization that certain patient demographic parameters may be expected to influence optimal settings for the fingerprint vectors (e.g. the thickness of a fat layer may affect the direction in the complex plane of a fingerprint vector).

According to one or more examples, the system 8 may include a display or other sensory output device for communicating the derived signal components to a user of the system.

As noted above, the system may include means for further processing the extracted signal components to derive from them a corresponding signal for the true physiological phenomenon or parameter to which the extracted signal component corresponds. Examples may include for instance a breathing rate signal, pulse rate signal, vessel diameter, blood pressure, or any other physiological parameter or measurement or signal. The extracted parameters or measurement signals may optionally be displayed using a display output.

According to one or more examples the system 8 may be further adapted to perform a learning procedure for adjusting or improving the stored fingerprint vectors $\vec{P}$, based on characteristics or features of previously extracted signal components for a given patient. For example, different patients have different geometries associated with their bodies and these differing geometries mean that a single set of stored characteristic fingerprint vectors will not necessarily be optimally attuned to every individual patient.

For example, each patient has a slightly different internal and external body shape (e.g., the thickness of a fat layer, the amount of muscles, the location of the heart, the amount of lung inflation, the work of breathing).

Hence the system 8 may be configured with a learning algorithm permitting initial learning and/or updating of the fingerprint vectors (e.g. automatically and dynamically) in accordance with the particular patient at hand. Optimal fingerprint vectors may be identified for a specific patient according to this procedure. This may involve application of a learning or adjustment algorithm adapted to trial different fingerprint vectors, this resulting in an output score of the likelihood of the corresponding extracted signal component originating from a certain target physiological origin (e.g., pulse).

A preferred method for dynamically learning or updating the finger-print vectors would be to apply one of various known methods in the category called Independent Component Analysis (ICA). The methods in this category have never been used in the context of inductive sensing. It has been found by the inventors that these methods are extremely effective in dynamically identifying the optimal finger-print vectors.

In essence, ICA algorithms assume that the detected signals at the resonator circuit (of dimension n > 1, i.e., multiple signals detected simultaneously) are a linear mixture or combination of (underlying, yet unknown, to be determined) source signals of dimension m ≤ n. To explain this further, an example is now considered in which there are two source signals $a_1$(t) and $a_2$(t) and two detected signals $s_1$(t) and $s_2$(t). These two are related via

$$\begin{pmatrix} s_1(t) \\ s_2(t) \end{pmatrix} = \begin{bmatrix} w_{11} & w_{12} \\ w_{21} & w_{22} \end{bmatrix} \begin{pmatrix} a_1(t) \\ a_2(t) \end{pmatrix} \tag{42}$$

or in matrix notation

$$\vec{s}(t) = \underline{W}\vec{a}(t) \tag{43}$$

where the weightvector matrix $\underline{W}$ describes how sources $a_1$(t) and $a_2$(t) map or transform to the detected signals $s_1$(t) and $s_2$(t). Independent component analysis is concerned with determining matrix $\underline{W}$ based on the detected signals $\vec{s}(t)$ so

that the unknown underlying sources $a_1$(t) and $a_2$(t) may be obtained based on a measurement of signals $s_1$(t) and $s_2$(t).

In particular, it can be most useful to derive the inverse of the weightvector matrix, $\underline{W}^{-1}$, since this describes more directly how to construct the unknown source $\vec{a}(t)$ based on the detected signals $\vec{s}(t)$, i.e.,

$$\vec{a}(t) = \underline{W}^{-1}\vec{s}(t). \tag{44}$$

Independent component analysis may therefore be used to obtain matrix $\underline{W}^{-1}$ based on the detected signal $\vec{s}(t)$ in order to compute the underlying source signals $\vec{a}(t)$.

This ICA approach can be applied directly to the determination of the fingerprint vectors for embodiments of the present invention. In particular, there is a direct match between the form of equation (39) and of equation (44). Detected signals $s_1$(t) and $s_2$(t) can be given for example by the measured frequency and the amplitude of the inductive sensor (or real and imaginary parts respectively of the reflected inductance, or the reactance and resistance respectively of the loop antenna - see table 1 above for the different options) and the underlying sources to be reconstructed, sources $a_1$(t) and $a_2$(t), are different physiological signal sources picked up by the antenna, for example breathing, and pulse (or breathing and motion, or pulse and motion).

Although the equations above show matrices with two rows only, this is for illustration only, and the matrices may have more than two rows, i.e. *n*, for extracting more than two physiological source signals, i.e. *n* source signals a(t).

Furthermore, when applied for embodiments of the present invention, the rows in matrix $\underline{W}^{-1}$ correspond to the fingerprint vectors of the inductive sensing method discussed above in relation to Equation (39). Thus, the fingerprint vector $\vec{P}_x$ as described in Eq (39) is one of the rows in matrix $\underline{W}^{-1}$. The number of source signals to be extracted from a set of measured signals $\vec{s}(t)$ is the number of rows in matrix $\underline{W}^{-1}$. Using the nomenclature of "fingerprint" vectors employed above, the inverse matrix $\underline{W}^{-1}$ might be described as the "fingerprint matrix".

Thus, it can be seen that Independent Component Analysis is a category of signal processing methods that is concerned with deriving matrix $\underline{W}^{-1}$, and that this can be advantageously applied to the identification and/or extraction from a set of sensed inductive signals of two or more physiological signal sources comprised by the sensed signals. This may be used therefore according to embodiments of the present invention for dynamically determining unknown fingerprint vectors.

The method may be applied as an initial procedure, e.g. an initial learning or calibration procedure, before the inductive sensing system is used in practice in order to learn the fingerprint vectors (or more broadly, the relative magnitudes of the real and imaginary components of reflected inductance) associated with different physiological sources for a particular patient at hand. It may additionally or alternatively be used to update the fingerprint vectors (or relative magnitudes).

The methodology of independent component analysis has never been applied in the field of inductive sensing. It is a non-obvious development since, despite 50 years of research into inductive sensors, it has never been recognized that the frequency and the damping can be considered as two independent signals, whose relative values can be used to identify a particular physiological source that is their originator.

A popular method of ICA is known as Fast ICA, for example described in the paper [Hyvärinen, A., & Oja, E. (2000). Independent component analysis : algorithms and applications. Neural Networks, 13, 411-430.]. It uses an efficient approximation for the entropy of the underlying source signal $\vec{a}(t)$, and it has an effective iterative method to optimize $\underline{W}^{-1}$ in order to minimize the entropy (maximize the non-Gaussianity).

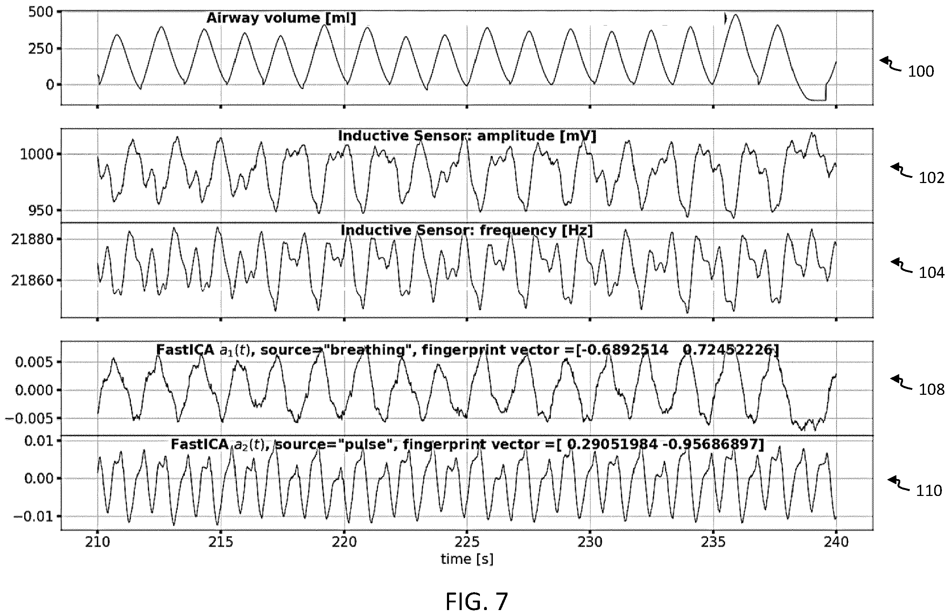

An example result of applying FastICA for detecting fingerprint vectors in accordance with one or more embodiments of the present invention is shown in Fig. 7. Fingerprint vectors are determined based on applying FastICA to the detected signals of amplitude and frequency of the resonator circuit current (as a function of time). The identified fingerprint vectors effectively decompose the detected composite (mixed) signals (amplitude and frequency) into the underlying components (or sources), corresponding to signals generated by breathing and pulse (for example).

In Fig. 7, the detected signals 102, 104 were obtained from an inductive sensor placed at the sternum of a volunteer who is breathing relatively fast (approximately 36 breaths per minute - see airway volume signal 100). Both the inductive sensor amplitude (signal 102) and frequency (signal 104) are recorded. The underlying physiological signal sources of pulse and breathing (signals 108 and 110 respectively) are extracted or reconstructed based on fingerprint vectors determined using the FastICA method discussed above (applied to the detected inductive sensing signals 102, 104).

The ICA method is thus very effective in pulling apart different physiological sources in an inductive sensor in which both the amplitude and the frequency are recorded simultaneously.

It is noted that although the above example is described in terms of detecting fingerprint vectors for different physiological sources, it can be understood as applicable more broadly to detecting the relative magnitude of real and imaginary components of reflected inductance for different physiological sources. The use of fingerprint vectors is one representation of this general concept discussed in this application.

More broadly, the learning procedure for the fingerprint vectors may be based on temporary use of any alternative method for separating sensed signal components from different physiological sources. These separated signals using the alternative method may according to some examples then be analyzed to determine their corresponding real and imaginary $L_r$ components, and the relative magnitudes of these components for the given signal source then stored in the dataset in the form for example of an updated fingerprint vector. Alternatively they may lead to determination of the fingerprint vectors directly, as in the ICA method above.

The alternative method may be any method which is currently known in the art for separating or distinguishing signal components from different physiological sources, such as based on different frequency compositions/spectra of the signal components, different magnitudes/amplitudes of the signal components, different quality measures of the signal components. Separating the signals based on these approaches may employ, by way of non-limiting example, use of any of: wavelets, signal frequency analysis, and/or system quality measures. ICA is one example approach for determining the different signal components or fingerprint vectors, but other approaches may in further examples alternatively be used.

In more detail, methods exist in the existing art of signal processing for separating sensed signal components having different physiological origins, based on certain a priori known characteristics associated with signals originating from those sources. For example, breathing rate is (most often) slower than the pulse rate, and hence the corresponding induction signals at the antenna will have correspondingly different frequencies. These signal components may hence in some examples be separated from one another based on frequency analysis of the overall composite signal sensed at the antenna.

Another existing method is based on analysis of a shape of the signal component waveform. For example, a typical breathing signal has a different shape than a pulse rate signal. Waveform based approaches may often make use of wavelets.

As discussed above, these methods employed in the state of the art can be misleading or unreliable, particularly in cases where certain pathologies exist which cause usually distinct signals to come closer to one another, such as when breathing rate is very high, or heart rate very slow. The use of fingerprint vectors is more reliable across all clinical scenarios.

The learning procedure, employing the known classical signal separation approaches discussed above, may in examples be performed in certain scenarios which are known to be clinically simple, and to not cause confusion or error in the separation of the signals. Once the learning procedure has updated the fingerprint vectors, the method according to embodiments of the present invention, based on use of relative magnitudes of real and imaginary $L_r$ components, may then be employed.

Simple clinical scenarios (during which the learning procedure may be employed) may (by way of non-limiting example) include any of the following: cases where motion artifacts are low (e.g. static patient; cases where there the patient does not have apnea; cases where these is no heart arrhythmias; cases where breathing rates and pulse rates are well separated; cases where the pulse waveform is not abnormal; cases where the breathing signal has a normal waveform).

Examples of non-simple clinical scenarios, during which implementation of the learning procedure employing classical signal separation approaches may be avoided, and instead the improved approach of embodiments of the present invention may be used, include for example one or more of the following:

- Cases in which there is an episode of apnea. Here, there is intermittently no breathing signal at all. Particularly for instance in cases of neonatal patients, the pulse rate may also drop during an apnea. During the absence of a breathing signal, a classical signal separation algorithm may mistake a pulse rate for a breathing rate.
- Cases in which breathing frequency and pulse frequency are similar. A frequency analysis approach may in this case fail to distinguish the two signals.
- Cases in which there are motion artifacts (e.g. movement of the patient or the antenna during sensing). These motion artifacts cause noise in the signal which may interfere with reliable signal separation.

Common machine learning techniques may also be applied in certain examples to improve or update the fingerprint vectors. For example, a machine learning algorithm might be employed, trained to separate signal components from different physiological sources based on the discussed classical approaches (e.g. frequency or waveform analysis).

It is noted that although in the above outlined examples, the stored information indicative of relative magnitudes of $L_r$ real and imaginary components for different physiological sources are in the form of a vector $\vec{P}$, this is not essential. In other examples, the relative magnitudes themselves may be stored, simply in numerical form. In other examples, other measures related to or derived from or otherwise indicative of the relative magnitudes of the real and imaginary components could be used, i.e. a proxy of the relative magnitudes of the real and imaginary components of $L_r$.

In some examples, simply a complex phase or argument of the complex $L_r$ component expected to be induced at the antenna by a given physiological source may be stored.

In accordance with an advantageous set of embodiments, the system 8 is further configured with an additional advantageous functionality for adjusting a frequency of the generated excitation signals in order to increase orthogonality of the characteristic vectors $\vec{P}$ for the different physiological sources in the body. The theory underlying this option will now be outlined in detail.

It can be derived from the determined expression for the additional inductance component received at the resonator circuit (characteristic reflected inductance $\hat{L}_r$) derived above that the argument or phase of the induced inductance component for signals originating from different regions of the body vary depending upon the frequency of the applied excitation signals. Hence by changing the frequency of the applied excitation signals, the phase or argument of the corresponding $\hat{L}$ signal component for a given physiological region will change.

In particular, the depth dependency (in the z direction) of the phase of the induced $\hat{L}_r$ signal at the antenna is strongly frequency dependent. Thus by adjusting the frequency of the applied excitation signals, it is possible to adjust the relative phases (i.e. arguments) of the reflected inductance, $\hat{L}_r$, signals originating from different locations in the body.

This means that the relative directions of the complex $\hat{L}$ signals in the complex plane for signals from different physiological sources can be varied by adjusting the frequency of the applied excitation signals. An equivalent way of expressing this is that the relative magnitudes of real and imaginary components of the complex $\hat{L}_r$ signal for signals originating from different physiological sources vary depending upon the frequency of the applied excitation signals.

In addition, adjusting the frequency of the applied excitation signals can also lead to a resulting change in the relative phases or arguments of signals originating from different tissue types in the body (even where these tissue types are at the same general location in the body).

This arises from the fact that the phase of the induced eddy currents in the body may typically make relatively large jumps or discontinuities across tissue type boundaries. This may be understood for instance from equation (25) above which shows that the loss tangent $\delta \equiv$ -Arg $(-i\omega\sigma + \omega^2\varepsilon_0\chi_e)$ affects the relative phase of the produced signal. At low frequencies, all tissues will have $\delta$=90°. However, at some tissue-dependent critical frequency $\omega_c = \dfrac{\sigma}{\varepsilon_0\chi_e}$, the loss tangent will move closer to $\delta$=0°. Since every tissue has a different conductivity and permittivity, the critical frequency at which the loss tangent changes to a different value is strongly tissue dependent. Thus, by changing the frequency, the relative phase of the signal coming from different tissues in the body can be tuned.

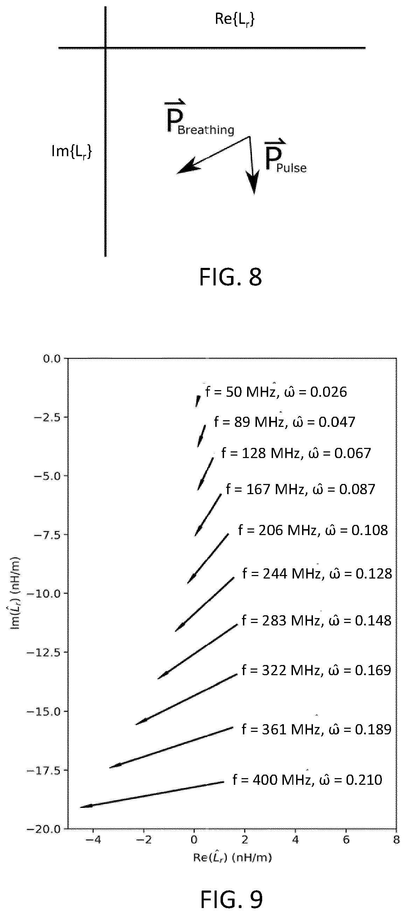

The frequency dependency of the phase of the induced reflected inductance component, $\hat{L}_r$, at the antenna for a given physiological source is illustrated in Fig. 8 and Fig. 9.

Fig. 8 schematically illustrates examples of the directions in the complex plane of two characteristic vectors $\vec{P}$ for two different physiological sources within the body. By way of example the two vectors are shown as corresponding to signals for breathing and for heart pulse. The x-axis corresponds to the real part of $\hat{L}_r$, the y-axis corresponds to the imaginary part of $\hat{L}_r$.

The start point of each vector corresponds to the characteristic reflected inductance at the start of the respective physiological cycle: deflated lung state and contracted heart state respectively for the breathing and pulse vectors. The end points correspond to the end of the respective physiological processes. Hence the difference between the two in each vector provides a representation of the respective physiological processes.

The end point minus the start point for each vector may be taken to correspond to the fingerprint vector in each case.

Fig. 9 illustrates different characteristics $L_r$ vectors $\vec{P}$ for different lung inflation levels of an example patient. Each arrow represents the vector for lung inflation for a different frequency of applied electromagnetic excitation signals. The x-axis corresponds to the real part of reflected inductance, $L_r$, the y-axis corresponds to the imaginary part of reflected inductance, $\hat{L}_r$. The arrow represents the change in reflected inductance as the lung inflates: the start point is $L_r$ when the lung is deflated, and the end point is $L_r$ when the lung is inflated. It can be seen that the start points vary for different frequencies and the end points vary for different frequencies. Hence the relative magnitudes of real and imaginary components of $L_r$ both at the start and end of the breathing process change depending upon frequency.

It can also be seen from the figure that the direction of the $L_r$ vector in the complex plane varies depending upon the chosen frequency of applied excitation signals.

By adjusting the frequency, the direction of the corresponding $L_r$ fingerprint vector can be changed.

The fingerprint vector may be taken to be either given by (relative magnitudes of real and imaginary components of) one or other of the start or end points, or by the change in the real and imaginary components over the lung inflation process.

According to an advantageous set of embodiments therefore, the frequency of the applied excitation signals may be adjusted in order to better orthogonalize the characteristic vectors $\vec{P}$ of the induced reflected inductance $L_r$ components at the antenna for the different physiological sources within the body. Greater orthogonality of these characteristic $L_r$ signals leads to more reliable and robust extraction and separation of signal components for the different physiological sources. It is therefore advantageous to adjust the applied signal frequencies to maximize orthogonality between the characteristic vectors.

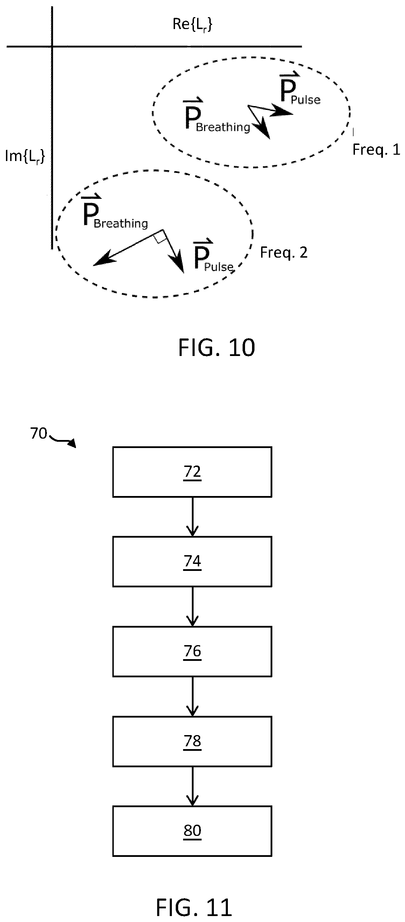

This process is schematically illustrated in Fig. 10 which illustrates relative directions of two example characteristic fingerprint vectors at a first frequency ("Freq. 1") and a second frequency ("Freq. 2"). As illustrated, at the first frequency the vectors are at a first relative phase separation from one another. By adjusting the frequency to the second frequency the vectors may be rendered orthogonal to one another within the complex plane.

To implement this functionality the system 8 may be configured with a frequency adjustment algorithm configured to detect, for instance in real time, a degree of orthogonality in the complex plane between vectors corresponding to complex $L_r$ components induced at the resonator circuit 10 for different physiological sources in the body.

A feedback loop may be implemented to the signal generation means (e.g. an oscillator), whereby the frequency of the generated electromagnetic excitation signals is adjusted to improve orthogonality, with the frequency adjustment guided or informed by the frequency feedback signal. The algorithm thereby adjusts the free space frequency of the oscillator to better orthogonalize the characteristic vectors of the reflected inductance signals for the different physiological sources.

The frequency adjustment may be based on temporary use of an alternative method for separating sensed signal components from different physiological sources. These separated signals using the alternative method can then be analyzed to determine their corresponding real and imaginary $L_r$ components, and hence to determine the corresponding fingerprint vectors for each signal component.

These alternative methods may be employed in conjunction with frequency adjustment to detect how fingerprint vector directions are changing as frequency is adjusted. A degree of orthogonality between the new set of fingerprint vectors for all received signal components may then be determined at each new frequency. This can be used to inform how to further adjust frequency to maximize orthogonality. A feedback loop can be implemented.

Additionally or alternatively to adjusting the frequency to increase orthogonality, according to one or more embodiments, the resonator circuit antenna may be driven (excited) at a plurality of frequencies quasi-simultaneously, for example 2 or 3 frequencies in a quasi-simultaneous manner. This can be done for example by multiplexing the frequency, i.e. alternating or cycling the frequency successively between a set of two or more frequencies, for example exciting the loop at a first frequency, and then at a second frequency, etc., and then returning back to said first frequency. This allows for measurement of inductive sensor signals at multiple different excitation frequencies quasi-simultaneously.

This frequency switching preferably is performed at a high frequency, e.g. at a sufficiently high frequency such that the Nyquist sample frequency for each the recorded frequencies is larger than the information bandwidth of the underlying physiological sources. The information bandwidth of typical source signals is at least about 5 Hz for pulse and breathing. Hence, in preferred embodiments, the minimum frequency at which the whole frequency-selection cycle should repeat itself is approximately 10 Hz.

Thus, switching the frequency of the drive signal to the antenna between two or more frequency settings (e.g. completing the full frequency cycle of multiple frequencies within at most approximately every 1/10 Hz = 0.1 seconds) would permit measurement of inductive sensor signals at multiple frequencies quasi-simultaneously. For signals representative of tissue motion, the information bandwidth may be larger and in this case a higher switching frequency may be preferable. The method of quickly switching between a plurality of different frequency settings may be referred to as time multiplexing of the drive or excitation frequency of the resonator circuit or antenna.

Recording inductive sensing signals at multiple frequencies in a quasi-simultaneous manner may be beneficial in particular in the context of a procedure for dynamically determining or learning or updating the fingerprint vectors (or more broadly the relative real and imaginary components of reflected inductance for different physiological sources). This is because these methods may perform poorly when the number of underlying physiological sources exceeds the number of detected signals. This can often be the case for inductive sensing, since sources such as "motion", "pulse", "breathing volume", and "breathing effort" may all be present at the same time. In this example, four different sources would be present, which is more than the 2 signals typically recorded (e.g. amplitude and frequency of the resonator circuit current) for an inductive sensor that operates at a single frequency.

This problem can be at least partially resolved by measuring the inductive sensor received signals or readings at multiple frequencies in the quasi-simultaneous manner as described above (i.e. time-multiplexing the frequency). By way of example, the drive frequency of the antenna may be alternated or multiplexed between two frequencies, e.g. 150 MHz and 250 MHz. If this is done, with the two inductive signals (e.g. of frequency and amplitude) measured for each frequency each duty cycle, this leads to a total of four independent detected measurement signals for each multiplexing duty cycle: "resonator amplitude at 150 MHz", "resonator frequency at 150 MHz", "resonator amplitude at 250 MHz", "resonator frequency at 250 MHz". Each of these is a (statistically) independent signal, and thus the collection of each extra signal adds genuine extra information. Their independence can be shown and understood from the physics of induction: different frequencies will induce eddy currents in the body of different geometries and/or positions, leading to return signals which are statistically independent. The fact that these signals are independent permits the improved determination of the four independent fingerprint vectors.

By way of example, the inductive sensor might be operated in a multiplexed manner with two multiplexed frequencies: 150 MHz and 250 MHz. As discussed above, this permits for example four detected signals to be measured. The FastICA method (see description above) might then be applied to this set of four signals to thereby obtain the corresponding four fingerprint vectors associated with the different physiological signal sources.

Preferably, the number of detected signals should match or exceed the number of physiological signal sources being received at the antenna at the same time. This ensures the FastICA method works most accurately for example. Thus, where frequency multiplexing is used, the number of multiplexed frequencies in each duty cycle is preferably equal to the number of physiological signal sources divided by two.

Although ICA is discussed above, the alternative method for separating the signal components may be any method which is currently known in the art for separating or distinguishing signal components from different physiological sources, such as based on different frequency compositions/spectra of the signal components, different magnitudes/amplitudes of the signal components, different quality measures of the signal components. Separating the signals based on these approaches may employ, by way of non-limiting example, use of any of: wavelets, signal frequency analysis, and/or system quality measures. These alternative methods, and use of them for detecting fingerprint vectors of signals from different physiological sources, has been discussed in detail above.

The stored fingerprint vectors in the dataset may be updated at the same time as the frequency is adjusted (and thus at the same time as the true physical $L_r$ vectors are changing), such that the extraction of the different signal components remains accurate.

To facilitate the above frequency adjustment feature, according to one set of examples, the system 8 may be provided having a signal generation means 14 which comprises a frequency-tunable oscillator circuit. This may be implemented in a number of different ways.

According to one approach, a bank of capacitors may be provided, each having high-Q switches to facilitate connection or disconnection of the capacitors to the resonator. By controlling the switches, the total capacitance of the resonator may be tuned, and thereby the frequency at which the excitation signals are generated also tuned.

According to one particular set of examples, the system may be configured in at least one control mode to switch the frequency of the signal generation means (e.g. the oscillator) between different frequencies at high speed. This switching may be done at frequencies for instance in the order of between 5-150 Hz. In this way, all of the different physiological sources in the body are probed at multiple different frequencies in a short space of time (e.g. quasi-simultaneously). In this way, the $L_r$ signal components can be sensed at many different frequencies providing a greater volume of information. By combining the fingerprint vectors from many frequencies, the signal separation may be rendered even more reliable.

Embodiments have been discussed above which involve accessing a dataset storing, for signals from a plurality of different known physiological sources within said body, information indicative of characteristic relative magnitudes of real and imaginary inductance components added to a resonator circuit by a signal received from said physiological source. However, use of such a dataset is not essential.

By way of example, according to a further set of embodiments, the extraction of the individual signal components may be based on application of an Independent Component Analysis procedure to the detected measures indicative of real and imaginary inductance components. In particular, the independent component analysis may be applied in an 'on-line' or real time manner to signals being sensed at the antenna, such that different physiological signal components are extracted from sensed measurement signals in real time using ICA. In this approach, a reference dataset of stored relative real and imaginary signal components for different physiological sources is not needed.

Application of an independent component analysis (ICA) procedure to received measurement signals has already been described above in relation to determination of fingerprint vectors for different physiological signal sources. However, the same ICA procedure may be applied in an on-line or real-time manner to incoming signals to determine and extract the different component physiological signals without a need for fingerprint vectors, or at least without the need for storing fingerprint vectors. The ICA method may simply by applied continuously to sensed signals (e.g. $s_1$, $s_2$), representative of measured additional real and imaginary inductance components added to the resonator circuit as a function of time. The weightvector matrix, W, may be derived (see above description) in real time, and this used to extract the physiological signal components, ai, $a_2$,...etc. A fingerprint vector might be derived and applied in real time (but not stored) or the weightvector matrix might be applied directly without a fingerprint vector being explicitly extracted. As the ICA procedure has already been described in detail above, it will not be described again here. The same procedure as discussed above may be applied to measured signals in real-time with their receipt and the component physiological signals derived in real time.

According to a further aspect of the invention, there is provided an inductive sensing method. An example method is shown in Fig. 11.

The method 70 based on sensing electromagnetic signals returned from a body responsive to propagation of electromagnetic excitation signals into said body.

The method comprises propagating 72 electromagnetic excitation signals into a body using a resonator circuit,

EP 3 897 377 B1

the resonator circuit comprising a loop antenna and an electrically coupled capacitor.

The method further comprises sensing 74 said returned signals from the body using the loop antenna, based on detecting variations in electrical characteristics of the resonator circuit.

The method further comprises the following steps for extracting from the sensed returned signals signal components from different physiological sources:

detecting 76 from the sensed returned signals measures indicative of both a real and an imaginary part of an additional inductance component induced in the resonator circuit by the received electromagnetic signals,

extracting 80 from the returned electromagnetic signals one or more individual signal components from different known physiological sources, based on use of the detected measures indicative of real and imaginary inductance components, the extracting being based on relative magnitudes of said detected real and imaginary inductance components added to the resonator circuit by the returned signals.

According to one set of embodiment, the method comprises

accessing 78 a dataset which stores, for signals from a plurality of different known physiological sources within said body, information indicative of characteristic relative magnitudes of real and imaginary inductance components added to a resonator circuit by a signal received from said physiological source; and

extracting 80 from the returned electromagnetic signals one or more individual signal components from different known physiological sources, based on use of the detected measures indicative of real and imaginary inductance components, and based on consulting said dataset.

Embodiments of the invention relate to an inductive sensing system and inductive sensing method.

Inductive sensors have the potential to become a highly valuable technology in the field of patient monitoring, in particular because biometric signals are relatively motion tolerant (meaning they are relatively unaffected by movement of the patient or of the measurement sensor). Possible applications advantageously include wearable patient monitors, use for motion tolerant respiration measurements, contactless patient monitoring, and spot-check measurements.

In preferred embodiments, an antenna is provided having a single loop (single winding), though this is not essential. A single loop winding provides benefits in terms of signal strength since parasitic capacitive coupling between windings is reduced. The same single loop antenna is used to both generate the excitation signals and to sense the returned signals. The returned signals are sensed simultaneously with signal generation by detecting changes in the electrical characteristics of the resonator circuit while the signals are being generated (and returned signals are being received at the antenna). A combined sensing and generating antenna allows for provision of high-quality sensing signals as well as providing benefits of low-cost, low complexity, and low-power.

Embodiments of the invention are based on sensing a real and imaginary part of an additional inductance component (reflected inductance) induced at the antenna 12 by received (returned) electromagnetic signals from the body. These parts can be detected by sensing changes in the frequency of the resonator circuit current and changes in the amplitude of the signal generator (e.g. oscillator) or of the resonator circuit current.

A wide range of potential applications for embodiments of the invention exist. By way of non-limiting example, applications include: patient monitoring; telemetry; spot-check monitoring; implementation in wearable devices (e.g. chest patches or wrist worn devices); neonatal monitoring; sleep monitoring; obstetrical monitoring; use for mattress-based sensors. A controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A

28

single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An inductive sensing system (8) for sensing electromagnetic signals returned from a body responsive to application of electromagnetic excitation signals to said body, the system comprising:

   a resonator circuit (10) comprising a loop antenna (12);
   a signal generation means (14) adapted to excite the loop antenna to generate the electromagnetic excitation signals,
   a signal sensing means (20) adapted to sense said returned signals from the body using the loop antenna, based on detecting variations in electrical characteristics of the resonator circuit; and
   a controller, wherein the controller is adapted to:

      detect from the sensed returned signals measures indicative of both a real and an imaginary part of an additional inductance component induced in the resonator circuit by the received electromagnetic signals, and **characterised by**
      using the detected measures indicative of real and imaginary inductance components, extract from the returned electromagnetic signals one or more individual signal components corresponding to different respective physiological sources, the extraction being based on relative magnitudes of said detected real and imaginary inductance components added to the resonator circuit by the returned signals.

2. The inductive sensing system according to claim 1, wherein the extraction of the individual signal components is based on application of an Independent Component Analysis procedure to the detected measures indicative of real and imaginary inductance components.

3. The inductive sensing system according to claim 1, wherein the system is adapted to:

   access a dataset which stores, for signals from a plurality of different known physiological sources within said body, information indicative of characteristic relative magnitudes of real and imaginary inductance components added to a resonator circuit by a signal received from said physiological source; and
   using the detected measures indicative of real and imaginary inductance components, and based on consulting said dataset, extract from the returned electromagnetic signals the one or more individual signal components corresponding to different respective known physiological sources.

4. The inductive sensing system (8) as claimed in claim 3, wherein the stored information for each physiological source takes the form of a vector or point in a complex plane, the vector or point representative of an additional inductance component added to a resonator circuit by a signal received from the respective physiological source.

5. The inductive sensing system, as claimed in claim 4, wherein said characteristic relative magnitudes represented by the stored information are weighted so as to provide a multiplicative mapping between the detected measures indicative of the real and imaginary parts of the additional sensed inductance component and an extracted signal component for a given physiological source.

6. The inductive sensing system (8) according to any of claims 4-5, wherein said stored information for each physiological source comprises a vector having:

   a first vector component indicative of a ratio between a physiological parameter related to the physiological source and a measure indicative of the real part of an additional inductance component added to a resonator circuit by a signal received from said physiological source, and
   a second vector component indicative of a ratio between a physiological parameter related to said physiological source and a measure indicative of the imaginary part of an additional inductance component added to a resonator circuit by a signal received from said physiological source.

7. The inductive sensing system (8) as claimed in any of claims 4-6, wherein extracting a given signal component comprises taking an inner product of a corresponding stored vector and a further vector, the further vector representative of said measures indicative of real and imaginary parts of the sensed returned electromagnetic signal(s), and
optionally wherein a low pass filter is applied to components of said further vector in advance of performing the inner product in order to remove a baseline and/or a band-pass filter is applied to said further vector in advance of performing the inner product.

8. The inductive sensing system (8) as claimed in any of claims 1-7, wherein

the signal sensing means is adapted to detect said measure indicative of the real part of the additional inductance component based on detecting a change in a reactance of the resonator circuit and/or based on detecting changes in a frequency of a current in the resonator circuit; and/or
the signal sensing means is adapted to detect said measure indicative of the imaginary part of the additional inductance component based on detecting a change in an electrical resistance of the antenna and/or based on detecting changes in an amplitude of a current in the resonator circuit.

9. The inductive sensing system (8) as claimed in any of claims 4-8, wherein

the signal generation means is adapted to excite the resonator to generate excitation signals having a radial frequency $\omega$, and
wherein the controller is adapted in at least one control mode to perform an adjustment of said radial frequency from a first frequency to a second frequency, to thereby increase a degree of orthogonality between respective vectors formed in the complex plane by the additional induced inductance components added to the resonator circuit by signals received from the different respective physiological sources.

10. The inductive sensing system (8) as claimed in claim 9, wherein the system is further adapted to update said stored information for each physiological source to reflect changes induced in the real and imaginary components of the additional induced inductance components for the different physiological sources by said frequency adjustment.

11. The inductive sensing system (8) as claimed in any of claims 3-10, wherein the system is adapted to perform a learning procedure comprising determining or updating the stored information for each of the physiological sources.

12. The inductive sensing system as claimed in claim 11, wherein the learning procedure comprises performing an independent component analysis, ICA, procedure upon the sensed returned signals, for example performing an ICA procedure upon signals representative of said real and imaginary parts of the additional inductance component added to the resonator circuit by the returned signals.

13. The inductive sensing system as claimed in any of claims 1-12, wherein the system is adapted to drive the antenna with a plurality of drive frequencies in a time-multiplexed manner and to sense the returned signals at the antenna at each of said drive frequencies.

14. The inductive sensing system (8) as claimed in any of claims 1-13, wherein the system includes signal processing means configured to process the extracted one or more signal components and derive one or more physiological parameter measurements based on the signal component(s).

15. An inductive sensing method (70) based on sensing electromagnetic signals returned from a body responsive to application of electromagnetic excitation signals to said body, the method comprising:

applying (72) electromagnetic excitation signals to a body using a resonator circuit, the resonator circuit comprising a loop antenna;
sensing (74) said returned signals from the body using the loop antenna, based on detecting variations in electrical characteristics of the resonator circuit;
detecting (76) from the sensed returned signals measures indicative of both a real and an imaginary part of an additional inductance component induced in the resonator circuit by the received electromagnetic signals; and
**characterised by**

extracting (80) from the returned electromagnetic signals one or more individual signal components from different known physiological sources, based on use of the detected measures indicative of real and imaginary inductance components, the extracting being based on relative magnitudes of said detected real and imaginary inductance components added to the resonator circuit by the returned signals, wherein a controller is adapted to perform the detecting (76) and extracting (80).

**Patentansprüche**

1. Induktives Erfassungssystem (8) zum Erfassen von elektromagnetischen Signalen, die von einem Körper in Reaktion auf Anlegen von elektromagnetischen Anregungssignalen an den Körper zurückgegeben werden, wobei das System Folgendes umfasst:

eine Resonatorschaltung (10), die eine Schleifenantenne (12) umfasst;
ein Signalerzeugungsmittel (14), das so angepasst ist, dass es die Schleifenantenne anregt, um die elektromagnetischen Anregungssignale zu erzeugen,
ein Signalerfassungsmittel (20), das so angepasst ist, dass es die vom Körper zurückgegebenen Signale unter Verwendung der Schleifenantenne basierend auf Detektieren von Variationen in elektrischen Eigenschaften der Resonatorschaltung erfasst; und
eine Steuereinheit, wobei die Steuereinheit so angepasst ist, dass sie:

aus den erfassten zurückgegebenen Signalen Messungen detektiert, die sowohl einen realen als auch einen imaginären Teil einer zusätzlichen Induktivitätskomponente angeben, die durch die empfangenen elektromagnetischen Signale in der Resonatorschaltung induziert wird, und **gekennzeichnet durch**,
unter Verwendung der detektierten Messungen, die die realen und imaginären Induktivitätskomponenten angeben, Extrahieren aus den zurückgegebenen elektromagnetischen Signalen von einer oder mehreren einzelnen Signalkomponenten, die jeweils unterschiedlichen physiologischen Quellen entsprechen, wobei die Extraktion auf den relativen Größen der detektieren realen und imaginären Induktivitätskomponenten basiert, die durch die zurückgegebenen Signale zur Resonatorschaltung hinzugefügt werden.

2. Induktives Erfassungssystem nach Anspruch 1, wobei die Extraktion der einzelnen Signalkomponenten auf der Anwendung einer Prozedur zur unabhängigen Komponentenanalyse auf die detektierten Messungen basiert, die reale und imaginäre Induktivitätskomponenten angeben.

3. Induktives Erfassungssystem nach Anspruch 1, wobei das System so angepasst ist, dass es:

auf einen Datensatz zugreift, der für Signale aus einer Vielzahl verschiedener bekannter physiologischer Quellen innerhalb des Körpers Informationen speichert, die die charakteristischen relativen Größen realer und imaginärer Induktivitätskomponenten angeben, die einer Resonatorschaltung durch ein von der physiologischen Quelle empfangenes Signal hinzugefügt werden; und
unter Verwendung der detektierten Messungen, die reale und imaginäre Induktivitätskomponenten angeben, und basierend auf der Abfrage des Datensatzes die eine oder die mehreren einzelnen Signalkomponenten aus den zurückgegebenen elektromagnetischen Signalen extrahiert, die jeweils unterschiedlichen bekannten physiologischen Quellen entsprechen.

4. Induktives Erfassungssystem (8) nach Anspruch 3, wobei die gespeicherten Informationen für jede physiologische Quelle die Form eines Vektors oder Punkts in einer komplexen Ebene annehmen, wobei der Vektor oder Punkt eine zusätzliche Induktivitätskomponente darstellt, die einer Resonatorschaltung durch ein von der jeweiligen physiologischen Quelle empfangenes Signal hinzugefügt wird.

5. Induktives Erfassungssystem nach Anspruch 4, wobei die durch die gespeicherten Informationen dargestellten charakteristischen relativen Größen gewichtet werden, um eine multiplikative Abbildung zwischen den detektierten Messungen, die den realen und imaginären Teil der zusätzlich erfassten Induktivitätskomponente angeben, und einer extrahierten Signalkomponente für eine gegebene physiologische Quelle bereitzustellen.

6. Induktives Erfassungssystem (8) nach einem der Ansprüche 4-5, wobei die gespeicherten Informationen für jede physiologische Quelle einen Vektor umfassen, der Folgendes aufweist:

eine erste Vektorkomponente, die ein Verhältnis zwischen einem physiologischen Parameter, der sich auf die physiologische Quelle bezieht, und einer Messung angibt, die den realen Teil einer zusätzlichen Induktivitätskomponente angibt, die einer Resonatorschaltung durch ein von der physiologischen Quelle empfangenes Signal hinzugefügt wird, und

eine zweite Vektorkomponente, die ein Verhältnis zwischen einem physiologischen Parameter, der sich auf die physiologische Quelle bezieht, und einer Messung angibt, die den imaginären Teil einer zusätzlichen Induktivitätskomponente angibt, die einer Resonatorschaltung durch ein von der physiologischen Quelle empfangenes Signal hinzugefügt wird.

7. Induktives Erfassungssystem (8) nach einem der Ansprüche 4-6, wobei Extrahieren einer gegebenen Signalkomponente Nehmen eines inneren Produkts aus einem entsprechenden gespeicherten Vektor und einem weiteren Vektor umfasst, wobei der weitere Vektor die genannten Messungen darstellt, die den realen und imaginären Teil des bzw. der erfassten zurückgegebenen elektromagnetischen Signale angeben, und
wobei optional vor Durchführen des inneren Produkts ein Tiefpassfilter auf die Komponenten des weiteren Vektors angewendet wird, um eine Basislinie zu entfernen, und/oder vor Durchführen des inneren Produkts ein Bandpassfilter auf den weiteren Vektor angewendet wird.

8. Induktives Sensorsystem (8) nach einem der Ansprüche 1-7, wobei

das Signalerfassungsmittel so angepasst ist, dass es die Messung detektiert, die den realen Teil der zusätzlichen Induktivitätskomponente angibt, basierend auf Detektieren einer Änderung der Reaktanz der Resonatorschaltung und/oder basierend auf Detektieren von Änderungen einer Frequenz eines Stroms in der Resonatorschaltung; und/oder
das Signalerfassungsmittel so angepasst ist, dass es die Messung detektiert, die den imaginären Teil der zusätzlichen Induktivitätskomponente angibt, basierend auf Detektieren einer Änderung des elektrischen Widerstands der Antenne und/oder basierend auf Detektieren von Änderungen der Amplitude eines Stroms in der Resonatorschaltung.

9. Induktives Erfassungssystem (8) nach einem der Ansprüche 4-8, wobei

das Signalerfassungsmittel so angepasst ist, dass es den Resonator anregt, um Anregungssignale zu erzeugen, die eine Radialfrequenz $\omega$ aufweisen, und
wobei die Steuereinheit so angepasst ist, dass sie in mindestens einem Steuermodus eine Anpassung der Radialfrequenz von einer ersten Frequenz auf eine zweite Frequenz durchführt, um dadurch einen Orthogonalitätsgrad zwischen jeweiligen Vektoren zu erhöhen, die in der komplexen Ebene durch die zusätzlichen induzierten Induktivitätskomponenten gebildet werden, die der Resonatorschaltung durch Signale hinzugefügt werden, die von den jeweiligen unterschiedlichen physiologischen Quellen empfangen werden.

10. Induktives Erfassungssystem (8) nach Anspruch 9, wobei das System weiter so angepasst ist, dass es die gespeicherten Informationen für jede physiologische Quelle aktualisiert, um durch die Frequenzanpassung induzierte Änderungen in den realen und imaginären Komponenten der zusätzlichen induzierten Induktivitätskomponenten für die verschiedenen physiologischen Quellen widerzuspiegeln.

11. Induktives Erfassungssystem (8) nach einem der Ansprüche 3-10, wobei das System so angepasst ist, dass es eine Lernprozedur durchführt, die Bestimmen oder Aktualisieren der gespeicherten Informationen für jede der physiologischen Quellen umfasst.

12. Induktives Erfassungssystem nach Anspruch 11, wobei die Lernprozedur Durchführen einer Prozedur einer unabhängigen Komponentenanalyse, ICA, an den erfassten zurückgegebenen Signalen umfasst, beispielsweise Durchführen einer ICA-Prozedur an Signalen, die die realen und imaginären Teile der zusätzlichen Induktivitätskomponente darstellen, die der Resonatorschaltung durch die zurückgegebenen Signale hinzugefügt werden.

13. Induktives Erfassungssystem nach einem der Ansprüche 1-12, wobei das System so angepasst ist, dass es die Antenne mit einer Vielzahl von Antriebsfrequenzen in einer Zeitmultiplex-Weise antreibt und die zurückgegebenen Signale an der Antenne bei jeder der Antriebsfrequenzen erfasst.

14. Induktives Erfassungssystem (8) nach einem der Ansprüche 1-13, wobei das System Signalverarbeitungsmittel einschließt, die so konfiguriert sind, dass sie die extrahierte eine oder mehreren Signalkomponenten verarbeiten und basierend auf der bzw. den Signalkomponenten eine oder mehrere Messungen physiologischer Parameter ableiten.

15. Induktives Erfassungsverfahren (70), basierend auf Erfassen von elektromagnetischen Signalen, die von einem Körper in Reaktion auf Anlegen von elektromagnetischen Anregungssignalen an den Körper zurückgegeben werden, wobei das Verfahren Folgendes umfasst:

Anlegen (72) elektromagnetischer Anregungssignale an einen Körper unter Verwendung einer Resonatorschaltung, wobei die Resonatorschaltung eine Schleifenantenne umfasst;

Erfassen (74) der vom Körper zurückgegebenen Signale unter Verwendung der Schleifenantenne basierend auf Detektieren von Variationen in elektrischen Eigenschaften der Resonatorschaltung;

Detektieren (76) aus den erfassten zurückgegebenen Signalen von Messungen, die sowohl einen realen als auch einen imaginären Teil einer zusätzlichen Induktivitätskomponente angeben, die durch die empfangenen elektromagnetischen Signale in der Resonatorschaltung induziert wird, und **gekennzeichnet durch**

Extrahieren (80) aus den zurückgegebenen elektromagnetischen Signalen einer oder mehrerer einzelner Signalkomponenten aus unterschiedlichen bekannten physiologischen Quellen basierend auf Verwendung der detektierten Messungen, die reale und imaginäre Induktivitätskomponenten angeben, wobei das Extrahieren auf relativen Größen der detektierten realen und imaginären Induktivitätskomponenten basiert, die der Resonatorschaltung durch die zurückgegebenen Signale hinzugefügt werden, wobei eine Steuereinheit so angepasst ist, dass sie das Detektieren (76) und Extrahieren (80) durchführt.

## Revendications

1. Système de détection par induction (8) destiné à détecter des signaux électromagnétiques renvoyés d'un corps en réponse à l'application de signaux d'excitation électromagnétiques audit corps, le système comprenant :

un circuit résonateur (10) comprenant une antenne cadre (12) ;

un moyen de génération de signaux (14) adapté pour exciter l'antenne cadre afin de générer les signaux d'excitation électromagnétiques,

un moyen de détection de signaux (20) adapté pour détecter lesdits signaux renvoyés du corps en utilisant l'antenne cadre, sur la base de la détection de variations de caractéristiques électriques du circuit résonateur ; et

un dispositif de commande, dans lequel le dispositif de commande est adapté pour :

détecter à partir des signaux renvoyés détectés des mesures indicatives à la fois d'une partie réelle et d'une partie imaginaire d'une composante d'inductance supplémentaire induite dans le circuit résonateur par les signaux électromagnétiques reçus, et **caractérisé par**

en utilisant les mesures détectées indicatives de composantes d'inductance réelles et imaginaires, extraire des signaux électromagnétiques renvoyés une ou plusieurs composantes de signal individuelles correspondant à différentes sources physiologiques respectives, l'extraction étant basée sur les grandeurs relatives desdites composantes d'inductance réelles et imaginaires détectées ajoutées au circuit résonateur par les signaux renvoyés.

2. Système de détection par induction selon la revendication 1, dans lequel l'extraction des composantes de signal individuelles est basée sur l'application d'une procédure d'analyse en composantes indépendantes aux mesures détectées indicatives de composantes d'inductance réelles et imaginaires.

3. Système de détection par induction selon la revendication 1, dans lequel le système est adapté pour :

accéder à un ensemble de données qui stocke, pour des signaux provenant d'une pluralité de différentes sources physiologiques connues dans ledit corps, des informations indicatives de grandeurs relatives caractéristiques de composantes d'inductance réelles et imaginaires ajoutées à un circuit résonateur par un signal reçu de ladite source physiologique ; et

en utilisant les mesures détectées indicatives de composantes d'inductance réelles et imaginaires, et sur la base de la consultation dudit ensemble de données, extraire des signaux électromagnétiques renvoyés les une ou plusieurs composantes de signal individuelles correspondant à différentes sources physiologiques connues respectives.

4. Système de détection par induction (8) selon la revendication 3, dans lequel les informations stockées pour chaque source physiologique prennent la forme d'un vecteur ou d'un point dans un plan complexe, le vecteur ou le point étant représentatif d'une composante d'inductance supplémentaire ajoutée à un circuit résonateur par un signal reçu de la source physiologique respective.

5. Système de détection par induction, selon la revendication 4, dans lequel lesdites grandeurs relatives caractéristiques représentées par les informations stockées sont pondérées de manière à fournir une cartographie multiplicative entre les mesures détectées indicatives des parties réelles et imaginaires de la composante d'inductance détectée supplémentaire et une composante de signal extraite pour une source physiologique donnée.

6. Système de détection par induction (8) selon l'une quelconque des revendications 4-5, dans lequel lesdites informations stockées pour chaque source physiologique comprennent un vecteur présentant :

une première composante vectorielle indicative d'un rapport entre un paramètre physiologique lié à la source physiologique et une mesure indicative de la partie réelle d'une composante d'inductance supplémentaire ajoutée à un circuit résonateur par un signal reçu de ladite source physiologique, et
une seconde composante vectorielle indicative d'un rapport entre un paramètre physiologique lié à ladite source physiologique et une mesure indicative de la partie imaginaire d'une composante d'inductance supplémentaire ajoutée à un circuit résonateur par un signal reçu de ladite source physiologique.

7. Système de détection par induction (8) selon l'une quelconque des revendications 4-6, dans lequel l'extraction d'une composante de signal donnée comprend la prise en compte d'un produit interne d'un vecteur stocké correspondant et d'un autre vecteur, l'autre vecteur étant représentatif desdites mesures indicatives de parties réelles et imaginaires du ou des signaux électromagnétiques renvoyés détectés, et
éventuellement dans lequel un filtre passe-bas est appliqué aux composantes dudit autre vecteur avant de réaliser le produit interne afin de supprimer une ligne de base et/ou un filtre passe-bande est appliqué audit autre vecteur avant de réaliser le produit interne.

8. Système de détection par induction (8) selon l'une quelconque des revendications 1-7, dans lequel

le moyen de détection de signaux est adapté pour détecter ladite mesure indicative de la partie réelle de la composante d'inductance supplémentaire sur la base de la détection d'un changement d'une réactance du circuit résonateur et/ou sur la base de la détection de changements de fréquence d'un courant dans le circuit résonateur ; et/ou
le moyen de détection de signaux est adapté pour détecter ladite mesure indicative de la partie imaginaire de la composante d'inductance supplémentaire sur la base de la détection d'un changement de résistance électrique de l'antenne et/ou sur la base de la détection de changements d'amplitude d'un courant dans le circuit résonateur.

9. Système de détection par induction (8) selon l'une quelconque des revendications 4-8, dans lequel

le moyen de génération de signaux est adapté pour exciter le résonateur afin de générer des signaux d'excitation présentant une fréquence radiale ω, et
dans lequel le dispositif de commande est adapté dans au moins un mode de commande pour réaliser un ajustement de ladite fréquence radiale d'une première fréquence à une seconde fréquence, pour augmenter ainsi un degré d'orthogonalité entre des vecteurs respectifs formés dans le plan complexe par les composantes d'inductance induites supplémentaires ajoutées au circuit résonateur par des signaux reçus des différentes sources physiologiques respectives.

10. Système de détection par induction (8) selon la revendication 9, dans lequel le système est en outre adapté pour mettre à jour lesdites informations stockées pour chaque source physiologique afin de refléter les changements induits des composantes réelles et imaginaires des composantes d'inductance induites supplémentaires pour les différentes sources physiologiques par ledit ajustement de fréquence.

11. Système de détection par induction (8) selon l'une quelconque des revendications 3-10, dans lequel le système est adapté pour réaliser une procédure d'apprentissage comprenant la détermination ou la mise à jour des informations stockées pour chacune des sources physiologiques.

12. Système de détection par induction selon la revendication 11, dans lequel la procédure d'apprentissage comprend la réalisation d'une procédure d'analyse en composantes indépendantes, ICA, sur les signaux renvoyés détectés, par exemple la réalisation d'une procédure ICA sur des signaux représentatifs desdites parties réelles et imaginaires de la composante d'inductance supplémentaire ajoutée au circuit résonateur par les signaux renvoyés.

13. Système de détection par induction selon l'une quelconque des revendications 1-12, dans lequel le système est adapté pour piloter l'antenne avec une pluralité de fréquences de pilotage de manière multiplexée dans le temps et pour détecter les signaux renvoyés à l'antenne à chacune desdites fréquences de pilotage.

14. Système de détection par induction (8) selon l'une quelconque des revendications 1-13, dans lequel le système inclut des moyens de traitement de signal configurés pour traiter les une ou plusieurs composantes de signal extraites et dériver une ou plusieurs mesures de paramètres physiologiques sur la base de la ou des composantes de signal.

15. Procédé de détection par induction (70), basé sur la détection de signaux électromagnétiques renvoyés d'un corps, en réponse à l'application de signaux d'excitation électromagnétiques audit corps, le procédé comprenant :

l'application (72) de signaux d'excitation électromagnétiques à un corps en utilisant un circuit résonateur, le circuit résonateur comprenant une antenne cadre ;
la détection (74) desdits signaux renvoyés du corps en utilisant l'antenne cadre, sur la base de la détection de variations de caractéristiques électriques du circuit résonateur ;
la détection (76) à partir des signaux renvoyés détectés de mesures indicatives à la fois d'une partie réelle et d'une partie imaginaire d'une composante d'inductance supplémentaire induite dans le circuit résonateur par les signaux électromagnétiques reçus ; et **caractérisé par**
l'extraction (80) à partir des signaux électromagnétiques renvoyés d'une ou plusieurs composantes de signal individuelles provenant de différentes sources physiologiques connues, sur la base de l'utilisation des mesures détectées indicatives de composantes d'inductance réelles et imaginaires, l'extraction étant basée sur des grandeurs relatives desdits composantes d'inductance réelles et imaginaires détectées ajoutées au circuit résonateur par les signaux renvoyés, dans lequel un dispositif de commande est adapté pour réaliser la détection (76) et l'extraction (80).

FIG. 1

FIG. 2

FIG. 3

FIG. 4

FIG. 5

FIG. 6

FIG. 7

EP 3 897 377 B1

FIG. 8

FIG. 9

EP 3 897 377 B1

FIG. 10

FIG. 11

41

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018127482 A1

- US 2015374292 A1

**Non-patent literature cited in the description**

- **HYVÄRINEN, A.** ; **OJA, E.** Independent component analysis : algorithms and applications. *Neural Networks*, 2000, vol. 13, 411-430